# EUROPEAN PATENT APPLICATION

(11) **EP 4 159 846 A1**
(43) Date of publication of application: **05.04.2023**
(21) Application number: 21812651.4
(22) Date of filing: 26.05.2021
(51) Int. Cl.: C12N 5/10, C12N 15/12

(54) **HYPOIMMUNOGENIC CELLS**

(30) Priority: 26.05.2020 JP 2020091787
(71) Applicant: HEALIOS K.K., Tokyo 100-0006 (JP)
(72) Inventor: TAMURA, Kouichi, Tokyo 100-0006 (JP); KIMURA, Hironobu, Tokyo 100-0006 (JP); HOSOYA, Tomonori, Tokyo 100-0006 (JP); TSUNEYOSHI, Norihiro, Tokyo 100-0006 (JP)
(74) Representative: J A Kemp LLP
(86) International application number: PCT/JP2021/020096
(87) International publication number: WO 2021/241658

(57) **Abstract**

The present invention provides a highly functional hypoimmunogenic cell, namely, a hypoimmunogenic human cell
(1) lacking an endogenous gene encoding an α chain of human leukocyte antigen (HLA) class Ia,
(2) lacking an endogenous gene encoding HLA class II or an expression regulator thereof,
(3) containing an exogenous gene encoding an α chain of HLA class Ib,
(4) containing an exogenous gene encoding human PD-L1, and
(5) containing an exogenous gene encoding human PD-L2.

## Description

### [Technical Field]

The present disclosure relates to a genetically-modified human pluripotent stem cell having extremely low immunogenicity, and a production method of the cell.

### [Background Art]

Major histocompatibility antigen (MHC) is known as human leukocyte antigen (HLA) in humans and is expressed in most cells and tissues. HLA mainly consists of 6 gene loci antigens of A, B, C, DR, DQ, and DP, each of which is composed of a complex combination of dozens of different types (alleles), and there are tens of thousands of combinations thereof. HLA plays an important role in the immune system in the human body, and its main role is antigen presentation for self-recognition. When non-self cells or tissues are transplanted to another person (allotransplantation), this HLA is recognized as the most important antigen (exogenous substance) by immunocompetent cells such as cytotoxic T cells (CTL), and rejection is established and the graft cannot survive.

While many of the cell medicines currently on the market are autologous cell products, the use of allogeneic cells is considered essential for the spread of cell medicines, and it is necessary to clear the problem of immune rejection.

The Center for iPS Cell Research and Application, Kyoto University, tried to solve this problem by stocking several types of iPS cells established from HLA homozygous donors. As of 2019, four kinds of HLA-type iPS cells have already been produced, which are common in Japanese people, and it is said that the stock iPS cells can cover about 40% of the Japanese people. However, with this method, it is extremely difficult to prepare cells that cover all people, and a huge amount of money is required. Furthermore, the possibility of immune rejection cannot be completely eliminated.

On the other hand, attempts to produce hypoimmunogenic cells by deleting HLA gene have been made for a long time. For example, Cell Genesys, Inc. discloses genetically modified cells lacking at least one MHC antigen (Patent Literature 1). In addition, Morphogenesis, Inc. also discloses a method for producing human stem cells deficient in HLA-B gene and HLA-C gene (Patent Literature 2).

In addition, attempts to produce hypoimmunogenic pluripotent stem cells are progressing rapidly because genetic modification of cells can be performed easily and accurately due to the rapid spread and development of genome editing technology in recent years, or the number of companies entering regenerative medicine and cell medicine is increasing.

Universal Cells Inc. (acquired by Astellas Pharma Inc.) and the University of Washington are developing universal donor cells obtained by deleting B2M gene and RFXANK gene in pluripotent stem cells (Patent Literatures 3 and 4). In addition, the University of California has developed hypoimmunogenic iPS cells in which B2M gene and CIITA gene have been deleted and CD47 has been overexpressed (Non Patent Literature 1).

On the other hand, Harvard University discloses therapeutic cells obtained by deleting B2M gene or CIITA gene, or knocking in PD-L1 gene or HLA-G gene (Patent Literature 5). In addition, Kyoto University has produced human iPS cells in which only HLA-A and HLA-B genes are individually deficient and CIITA gene is deficient (Non Patent Literature 2).

As described, various hypoimmunogenic cells have been produced by deleting and introducing various genes including HLA gene. However, there are hardly cases where detailed analysis has been made as to how the deletion of which HLA gene influences the expression of other genes involved in immune rejection. In addition, there are hardly cases where more highly functional hypoimmunogenic cell is obtained by knocking in which gene is analyzed. Under such circumstances, there is still a demand for a highly functional hypoimmunogenic cell.

### [Citation List]

### [Patent Literature]

[PTL 1]
   WO 1995/017911
[PTL 2]
   WO 98/42838
[PTL 3]
   WO 2016/183041
[PTL 4]
   WO 2012/145384
[PTL 5]
   WO 2013/158292

### [Non Patent Literature]

[NPL 1]
   Tobias Deuse et al., Nature Biotechnology, volume 37, pages 252-258, 2019
[NPL 2]
   Huaigeng Xu et al., Cell Stem Cell, 24, 1-13, 2019

### [Summary of Invention]

### [Technical Problem]

A highly functional hypoimmunogenic cell is demanded.

### [Solution to Problem]

The inventors considered that the production of hypoimmunogenic cells to replace stock iPS cells can contribute to the development of allogeneic cell medicines, and investigated combinations of proteins involved in various immunorejections by using genome editing tools.

First, with the aim of avoiding immune responses by CTL, the inventors deleted the endogenous gene encoding each α-chain of HLA class Ia (HLA-A, HLA-B, and HLA-C) that binds to the T cell receptor (TCR) of CTL. In addition, in order to delete HLA class II that binds to the TCR of helper T cells, they deleted an endogenous gene encoding RFXANK, which is one of the transcriptional regulators of HLA class II gene. Cells deficient in each of these genes were considered not generally rejected by immunity. However, it was unexpectedly clarified that the expression of intact HLA class Ib (HLA-E) was also lost in the above-mentioned cells. Cells that do not express HLA class Ib are inconvenient for use as a cell source for transplantation because they are attacked by NK cells. Therefore, the need arose to introduce a gene encoding HLA class Ib.

In view of the above-mentioned need, the inventors introduced a gene encoding HLA class Ib, as well as each gene that encodes PD-L1 and PD-L2 that bind to PD-1 and PD-2 on CTL and act suppressively on CTL. Finally, they clarified that the HLA class I expression level can be improved by introducing a gene encoding the common light chain β2 microglobulin (B2M) of HLA class Ia and HLA class Ib.

As a result of these, it was found that hypoimmunogenic human pluripotent stem cells having high safety and retaining differentiation induction potency can be produced by, in a human pluripotent stem cell, deleting an endogenous gene encoding each α chain of HLA-A, HLA-B and HLA-C which are HLA class Ia molecules, deleting an endogenous gene encoding RFXANK, and introducing respective exogenous genes encoding PD-L1 and PD-L2, respective exogenous genes encoding each α chain of HLA-E and/or HLA-G, which are HLA class Ib molecules, and an exogenous gene encoding β2 microglobulin into the genome. Based on these findings, the inventors have conducted further intensive studies and completed the present invention.

Accordingly, the present invention provides the following.
[1] A hypoimmunogenic human cell
   (1) lacking an endogenous gene encoding an α chain of human leukocyte antigen (HLA) class Ia,
   (2) lacking an endogenous gene encoding HLA class II or an expression regulator thereof,
   (3) comprising an exogenous gene encoding an α chain of HLA class Ib,
   (4) comprising an exogenous gene encoding human PD-L1, and
   (5) comprising an exogenous gene encoding human PD-L2.
[2] The cell of [1], wherein the cell does not express endogenous HLA class Ib on a cell surface.
[3] The cell of [1] or [2], wherein the endogenous gene encoding the α chain of the HLA class Ia comprises an endogenous gene encoding an α chain of HLA-A, an endogenous gene encoding an α chain of HLA-B, and an endogenous gene encoding an α chain of HLA-C.
[4] The cell of any one of [1] to [3], wherein the endogenous gene encoding the HLA class II or an expression regulator thereof comprises the following (a) or (b):
   (a) an endogenous gene encoding an α chain and/or a β chain of HLA-DP, an endogenous gene encoding an α chain and/or a β chain of HLA-DQ, an endogenous gene encoding an α chain and/or a β chain of HLA-DR, an endogenous gene encoding an α chain and/or a β chain of HLA-DM, and an endogenous gene encoding an α chain and/or a β chain of HLA-DO,
   (b) an endogenous gene encoding human RFXANK, an endogenous gene encoding human RFX5, an endogenous gene encoding human RFXAP, and an endogenous gene encoding human CIITA.
[5] The cell of any one of [1] to [4], wherein the exogenous gene encoding the α chain of the HLA class Ib comprises an exogenous gene encoding an α chain of HLA-E and/or an exogenous gene encoding an α chain of HLA-G.
[6] The cell of any one of [1] to [5], comprising (6) an exogenous gene encoding human β2 microglobulin.
[7] The cell of any one of [1] to [6], comprising (7) a suicide gene.
[8] The cell of any one of [1] to [7], wherein the site containing the exogenous gene or suicide gene is a safe harbor region of the genome.
[9] The cell of [8], wherein the safe harbor region is an AAVS1 region, a CCR5 region, or a ROSA26 region.
[10] The cell of any one of [1] to [9], wherein the hypoimmunogenic human cell is a pluripotent stem cell or a differentiated cell thereof.
[11] A method for producing a hypoimmunogenic human cell, comprising the following steps:
   (i) a step of deleting an endogenous gene encoding an α chain of HLA class Ia of a human parental cell,
   (ii) a step of deleting an endogenous gene encoding HLA class II or an expression regulator thereof from the human parental cell,
   (iii) a step of introducing an exogenous gene encoding an α chain of HLA class Ib into the human parental cell,
   (iv) a step of introducing an exogenous gene encoding human PD-L1 into the human parental cell, and
   (v) a step of introducing an exogenous gene encoding human PD-L2 into the human parental cell.
[12] The method of [11], wherein the hypoimmunogenic human cell does not express endogenous HLA class Ib on a cell surface.
[13] The method of [11] or [12], wherein the endogenous gene encoding the α chain of the HLA class Ia comprises an endogenous gene encoding an α chain of HLA-A, an endogenous gene encoding an α chain of HLA-B, and an endogenous gene encoding an α chain of HLA-C.
[14] The method of any one of [11] to [13], wherein the endogenous gene encoding HLA class II or an expression regulator thereof comprises the following (a) or (b):
   (a) an endogenous gene encoding an α chain and/or a β chain of HLA-DP, an endogenous gene encoding an α chain and/or a β chain of HLA-DQ, an endogenous gene encoding an α chain and/or a β chain of HLA-DR, an endogenous gene encoding an α chain and/or a β chain of HLA-DM, and an endogenous gene encoding an α chain and/or a β chain of HLA-DO,
   (b) an endogenous gene encoding human RFXANK, an endogenous gene encoding human RFX5, an endogenous gene encoding human RFXAP, and an endogenous gene encoding human CIITA.
[15] The method of any one of [11] to [14], wherein the exogenous gene encoding the α chain of the HLA class Ib comprises an exogenous gene encoding an α chain of HLA-E and/or an exogenous gene encoding an α chain of HLA-G.
[16] The method of any one of [11] to [15], further comprising the following step:
   (vi) a step of introducing an exogenous gene encoding human β2 microglobulin into the human parental cell.
[17] The method of any one of [11] to [16], further comprising the following step:
   (vii) a step of introducing a suicide gene into the human parental cell.
[18] The method of any one of [11] to [17], wherein the site into which the exogenous gene or suicide gene is introduced is a safe harbor region of the genome.
[19] The method of [18], wherein the safe harbor region is an AAVS1 region, a CCR5 region, or a ROSA26 region.
[20] The method of any one of [11] to [19], wherein the human parental cell is a pluripotent stem cell or a differentiated cell thereof.

### [Advantageous Effects of Invention]

A hypoimmunogenic human pluripotent stem cell retaining differentiation induction potency can be acquired by, in a human pluripotent stem cell, deleting endogenous genes encoding each α chain of HLA-A, HLA-B and HLA-C which are of HLA class Ia, deleting endogenous genes encoding RFXANK which is a HLA class II transcription factor, introducing respective exogenous genes encoding PD-L1 and PD-L2 which are immune checkpoint proteins, and introducing exogenous genes encoding each α chain of HLA-E and/or HLA-G into the genome to complement the defective expression of endogenous HLA class Ib. Furthermore, the inventors have further found for the first time that the expression level of HLA class Ib can be improved by introducing an exogenous gene encoding B2M into the genome, and as a result, the hypoimmunogenicity of the cells can be improved beyond expectations. The inventors have successfully produced the hypoimmunogenic cells of the present invention based on these findings. In addition, it is possible to arbitrarily induce apoptosis by introducing a suicide gene into the genome. The hypoimmunogenic cells obtained by the present invention still maintain pluripotency and can be induced to differentiate into any cells.

The hypoimmunogenic cells obtained by the present invention can be used as a starting material for allogeneic cell medicines. Even if the cells obtained by differentiation induction of these cells are transplanted, they are not rejected by T cells or NK cells from the recipient side, or the degree of immune rejection is extremely low. In addition, the above-mentioned hypoimmunogenic cells suppress the activation of antigen-presenting cell groups such as B cells, macrophages, monocytes, and dendritic cells.

Therefore, these cells can be safely used for transplantation therapy and cell therapy.

### [Brief Description of Drawings]

[Fig. 1]
   A figure showing gene editing results of HLA class Ia-deficient iPS cell clone 2E1.
[Fig. 2]
   Figures showing the analysis results of the cell surface expression levels of HLA class I in clone 2E1 by a flow cytometry method.
[Fig. 3]
   Figures showing the analysis results of the cell surface expression levels of HLA-E in clone 2E1 by a flow cytometry method.
[Fig. 4]
   Figures showing the analysis results of the cell surface expression levels of undifferentiated markers in clone 2E1 by a flow cytometry method.
[Fig. 5]
   Figures showing the analysis results of the RNA expression patterns of clone 2E1 and the second candidate clone (2H3) .
[Fig. 6]
   A figure showing the gene editing results of HLA class Ia&II-deficient iPS cell clone 6B7.
[Fig. 7]
   Figures showing the analysis results of the cell surface expression levels of HLA class I in clone 6B7 by a flow cytometry method.
[Fig. 8]
   Figures showing the analysis results of the cell surface expression levels of HLA class II in clone 6B7 by a flow cytometry method.
[Fig. 9]
   Figures showing the analysis results of the cell surface expression levels of HLA-E in clone 6B7 by a flow cytometry method.
[Fig. 10]
   Figures showing the analysis results of the cell surface expression levels of undifferentiated markers in clone 6B7 by a flow cytometry method.
[Fig. 11]
   A figure showing the analysis results of the RNA expression pattern in clone 6B7.
[Fig. 12]
   Figures respectively showing A: analysis results of the urea synthesis amount of hepatocytes differentiated from clone 6B7, and B: analysis results of the angiogenic potential of vascular endothelial cells differentiated from clone 6B7.
[Fig. 13]
   Figures showing the analysis results of the cell surface expression levels of PD-L1, PD-L2, HLA-G, and B2M in iPS cell clone 9G11 in which HLA class Ia & II are deleted and PD-L1, PD-L2, HLA-G, B2M, and iCasp9 genes are introduced by a flow cytometry method.
[Fig. 14]
   Figures showing the analysis results of the cell surface expression levels of HLA-A, HLA-B, HLA-C, and HLA class II in clone 9G11 iPS cell-derived hematopoietic cells by a flow cytometry method.
[Fig. 15]
   Figures showing the analysis results of the cell surface expression levels of PD-L1, PD-L2, HLA-G and B2M in clone 9G11 iPS cell-derived hematopoietic cells by a flow cytometry method.
[Fig. 16]
   Figures showing the analysis results of the cell surface expression levels of undifferentiated markers in clone 9G11 by a flow cytometry method.
[Fig. 17]
   A figure showing the results of the karyotype analysis in clone 9G11.
[Fig. 18]
   A: a figure showing the cell morphology of hepatocytes differentiated from clone 9G11 and showing the analysis results of the urea synthesis amount thereof, and B: a figure showing the cell morphology of vascular endothelial cells differentiated from clone 6B7 and a figure showing the analysis results of the angiopoietic potential thereof.
[Fig. 19]
   Figures showing proliferation rates of T cells (CD4-positive cells and CD8-positive cells) against CD45-positive cells differentiated from clone 9G11.
[Fig. 20]
   A figure showing the cytotoxic activity of T cells (CD8-positive cells) against CD45-positive cells differentiated from clone 9G11.
[Fig. 21]
   A figure showing the cytotoxic activity of NK cells against clone 9G11.
[Fig. 22]
   A figure showing changes in cell viability after rapamycin treatment, in order to confirm the function of suicide gene of clone 9G11.
[Fig. 23]
   Figures showing that the forced expression of exogenous B2M gene increases the expression level of HLA-G gene in HLA class Ia&II-deficient iPS cells.

### [Description of Embodiments]

The contents of the present invention are described in detail below.

The present invention provides a hypoimmunogenic human cell having the following characteristics (1) to (5) (hereinafter the hypoimmunogenic human cell of the present invention):
(1) lacking an endogenous gene encoding an α chain of HLA class Ia,
(2) lacking an endogenous gene encoding HLA class II or an expression regulator thereof,
(3) comprising an exogenous gene encoding an α chain of HLA class Ib,
(4) comprising an exogenous gene encoding human PD-L1, and
(5) comprising an exogenous gene encoding human PD-L2.

The hypoimmunogenic human cell of the present invention is a cell that, when introduced into the body of a recipient, does not cause initiation of immunorejection reactions that normally occur when recognized as being non-self by the recipient, that is not attacked by recipient T cells or NK cells, that suppresses the activation of antigen-presenting cells, and that has extremely low reactivity even if immunorejection occurs. In other words, the hypoimmunogenic human cell of the invention is a cell that is immunologically tolerant to the recipient's immune system.

The hypoimmunogenic human cell of the present invention has acquired immune tolerance to the recipient's immune system by deleting the above-mentioned endogenous genes (1) and (2) and introducing the above-mentioned exogenous genes (3), (4), and (5). Therefore, the hypoimmunogenic human cell of the present invention can serve as a cell medicine for the recipient.

The hypoimmunogenic human cell of the present invention is not particularly limited as long as it permits deletion of the above-mentioned endogenous genes (1) and (2) and introduction of the above-mentioned exogenous genes (3), (4), and (5). Such cell includes pluripotent stem cells.

Examples of the pluripotent stem cell include embryonic stem cells (ES cells), induced pluripotent stem cells (iPS cells), embryonal carcinoma cells (EC cells), and embryonic germ cells (EG cells), with preference given to ES cell or iPS cell.

When the pluripotent stem cell is an ES cell, it can be produced by a method known per se. The methods for producing ES cells include, but are not limited to, for example, a method of culturing the inner cell mass at the human blastocyst stage (e.g., Manipulating the Mouse Embryo: A Laboratory Manual, Second Edition, Cold Spring Harbor Laboratory Press (1994)), a method of culturing an early embryo produced by somatic cell nuclear transplantation (Wilmut et al., Nature, 385, 810 (1997); Cibelli et al., Science, 280, 1256 (1998); valley, protein nucleic acid enzyme, 44, 892 (1999); Baguisi et al., Nature Biotechnology, 17, 456 (1999); Wakayama et al., Nature, 394, 369 (1998); Wakayama et al., Nature Genetics, 22, 127 (1999); Wakayama et al., Proc. Natl. Acad. Sci. USA, 96, 14984 (1999); RideoutIII et al., Nature Genetics, 24, 109 (2000)), and the like. In addition, ES cells can be obtained from specified institutions, and commercially available products can also be purchased. For example, human ES cell lines H1, H7, H9, H13 and H14 are available from the WiCell Research Institute in the United States; HES1 - 6 are available from the ES Cell International in Australia, SA002, SA181, and SA611 are available from Cellartis AB in Sweden, HUES1 - 17 are available from HUES Cell Facility in the United States, KhES-1 - KhES-5 are available from The Institute for Frontier Life and Medical Sciences, Kyoto University, and SEES1 - SEES7 are available from the National Center for Child Health and Development. When ES cells are produced by somatic cell nuclear transplantation, the type of somatic cells and the source from which somatic cells are collected are in accordance with the following case of iPS cell production.

When the pluripotent stem cells are iPS cells, iPS cells can be produced by introducing nuclear reprogramming substances into somatic cells. Somatic cells that can be used as starting materials for iPS cell production may be any cells other than human germ cells. Examples thereof include keratinizing epithelial cells (e.g., keratinized epidermal cells), mucosal epithelial cells (e.g., epithelial cells of tongue surface), exocrine gland epithelial cells (e.g., mammary gland cells), hormone-secreting cells (e.g., adrenal medullary cells), cells for metabolism/storage (e.g., hepatocytes), luminal epithelial cells constituting boundary surface (e.g., type I alveolar cells), vascularluminal epithelial cells (e.g., vascular endothelial cells), ciliated cells with transport capacity (e.g., airway epithelial cells), cells for extracellular matrix secretion (e.g., fibroblasts), contractile cells (e.g. smooth muscle cells), blood and immune system cells (e.g. T lymphocytes), cells related to senses (e.g. rod cells), autonomic nervous system neurons (e.g., cholinergic neurons), supporting cells of sensory organs and peripheral neurons (e.g. satellite cells), neurons and glia cells of the central nervous system (e.g., astroglial cells), pigment cells (e.g., retinal pigment epithelial cells), progenitor cells thereof (tissue progenitor cells), and the like. There is no particular limitation on the degree of cell differentiation, and both undifferentiated progenitor cells (also including somatic stem cells) and terminally differentiated mature cells can be similarly used as sources of somatic cells in the present invention. Examples of the undifferentiated progenitor cell include tissue stem cells (somatic stem cells) such as adipose-derived stromal (stem) cells, neural stem cells, hematopoietic stem cells, mesenchymal stem cells, dental pulp stem cells, and the like.

The nuclear reprogramming substances to be introduced into somatic cells for the production of iPS cells include combinations of various reprogramming genes that have been reported to date (e.g., WO2007/069666, Nature Biotechnology, 26, 101-106 (2008), Cell, 126, 663-676 (2006), Cell, 131, 861-872 (2007), Nat. Cell Biol., 11, 197-203 (2009), Nature, 451, 141-146 (2008), Science, 318, 1917-1920 (2007), Stem Cells, 26, 1998-2005 (2008), Cell Research (2008) 600-603, Nature 454: 646-650 (2008), Cell Stem Cell,2: 525-528(2008), WO2008/118820, Nat. Cell Biol., 11, 197-203 (2009), Nat. Cell Biol., 11, 197-203 (2009), Science, 324: 797-801 (2009)). In addition, the protein encoded by the above-mentioned reprogramming gene can also be introduced into somatic cells as a nuclear reprogramming substance (Cell Stem Cell, 4: 381-384(2009), Cell Stem Cell, doi:10.1016/j.stem.2009.05.005 (2009)).

Selection of iPS cell colony can be performed by a method using drug resistance and reporter activity as indices (Cell, 126, 663-676 (2006), Nature, 448, 313-317 (2007)) and a method by visual observation of morphology (Cell, 131, 861-872 (2007)). iPS cells can be confirmed using the expression of various ES cell-specific genes and teratoma formation as indices.

At present, there are various methods for producing iPS cells (iPSC), and the production method of iPSC established by Yamanaka et al. by introducing four factors of Oct3/4, Sox2, Klf4, and c-Myc into mouse fibroblasts (Takahashi K, Yamanaka S., Cell, (2006) 126: 663-676), as well as a production method of human cell-derived iPSC established by introducing similar four factors into human fibroblasts (Takahashi K, Yamanaka S., et al. Cell, (2007) 131: 861-872.), a production method of Nanog-iPSC established by introducing the above-mentioned four factors, and then selecting using Nanog expression as an index (Okita, K., Ichisaka, T., and Yamanaka, S. (2007). Nature 448, 313-317.), a production method of iPSC without using c-Myc (Nakagawa M, Yamanaka S., et al. Nature Biotechnology, (2008) 26, 101 - 106), a production method of iPSC established by introducing six factors by a virus-free method (Okita K et al. Nat. Methods 2011 May; 8(5):409-12, Okita K et al. Stem Cells. 31(3): 458-66.), and the like can also be used. In addition, the production method of iPSC established by Thomson et al. by introducing four factors of Oct3/4, Sox2, NANOG, and LIN28 (Yu J., Thomson JA. et al., Science (2007) 318: 1917-1920.), the production method of iPSC by Daley et al. (Park IH, Daley GQ. et al., Nature (2007) 451: 141-146), the production method of iPSC by Sakurada et al. (JP-A-2008/307007), and the like can also be used.

As induced pluripotent stem cell lines, any of various human iPSC lines established by NIH, RIKEN, Kyoto University, and the like can be used. For example, HiPS-RIKEN-1A strain, HiPS-RIKEN-2A strain, HiPS-RIKEN-12A strain, Nips-B2 strain, and the like of RIKEN, and 253G1 strain, 253G4 strain, 1201C1 strain, 1205D1 strain, 1210B2 strain, 1383D2 strain, 1383D6 strain, 201B7 strain, 409B2 strain, 454E2 strain, 606A1 strain, 610B1 strain, 648A1 strain, 1231A31 strain, FfI-01s04 strain, and the like of Kyoto University can be mentioned.

The hypoimmunogenic human cell of the present invention may be a cell differentiated from the aforementioned pluripotent stem cell obtained by deleting the endogenous genes in the above-mentioned (1) and (2) and introducing the exogenous genes in the above-mentioned (3), (4), and (5). Pluripotent stem cells can be differentiated into specific cells according to known methods. For example, pluripotent stem cells can be differentiated into T cells (WO 2016/076415 or WO 2017/221975), corneal epithelial cells (WO 2016/114285), cardiomyocytes (WO 2007/126077, WO 2016/049099, WO 2016/175303 or WO 2017/108705), pancreatic β cells (WO 2019/208788), hepatocytes (WO 2013/183571 or WO 2019/073951), skeletal muscle cells (WO 2010/008100, WO 2014/533491 or WO 2017/188458), retinal pigment epithelial cells (WO 2015/053375), and the like.

The hypoimmunogenic human cell of the present invention lacks an endogenous gene encoding α chain of HLA class Ia.

HLA class Ia is a transmembrane protein possessed by all nucleated cells and has some thousands or more of diverse polymorphisms. Thus, HLA class Ia expressed on the cell surface is very diverse for each individual. Such diversity plays the most important role in distinguishing between self and non-self. HLA class Ia presented on the cell surface is recognized by the T cell receptor (TCR) on the CTL surface. Cells presenting the recognized HLA class Ia are recognized as exogenous substances by CTL and eliminated by the immune system. Therefore, cells deficient in HLA class Ia expression can avoid recognition by CTL and thus are suitable as transplantation cell sources.

HLA class Ia is a dimer consisting of α chain encoded by each gene of HLA class Ia and β chain which is β2 microglobulin. The β2 microglobulin is a common component that associates with not only the α-chain of HLA class Ia, but also the α-chain of HLA class Ib. Deletion of the endogenous gene encoding β2 microglobulin to achieve defective expression of HLA class Ia has been done in other hypoimmunogenic cells. However, it is inconvenient because expression of HLA class Ib is also rendered defective. Therefore, in the hypoimmunogenic human cell of the present invention, the endogenous genes encoding the α chain of HLA class Ia are deleted so as to render only the expression of HLA class Ia defective.

As the endogenous genes encoding the α chain of HLA class Ia deficient in the hypoimmunogenic human cell of the present invention, at least one gene selected from the group consisting of an endogenous gene encoding α chain of HLA-A, an endogenous gene encoding α chain of HLA-B, and an endogenous gene encoding α chain of HLA-C, preferably, an endogenous gene encoding α chain of HLA-A, an endogenous gene encoding α chain of HLA-B, and an endogenous gene encoding α chain of HLA-C can be mentioned.

In the present specification, deletion of an endogenous gene means prevention of the production of a complete mRNA by disrupting or removing the endogenous gene.

As a specific means of deleting endogenous genes, a method including integrating, into the endogenous gene locus of the target cell by homologous recombination, a DNA chain having a DNA sequence constructed to inactivate the gene (hereinafter to be abbreviated as the targeting vector for gene deletion) which is obtained by isolating according to a conventional method a genomic DNA derived from a target cell in which the endogenous gene is to be deleted and, for example, (1) disrupting the functions of exon or promoter of the endogenous gene by inserting other DNA fragment (e.g., drug resistance gene, reporter gene, etc.) into the exon or promoter region, (2) cutting out all or part of the endogenous gene by using Cre-loxP system or Flp-frt system to make the gene defective, (3) inserting a stop codon into a protein coding region to disable translation of the complete protein, or (4) inserting a DNA sequence (e.g., poly A addition signal and the like) that terminates transcription of the gene into the transcribed region in order to disable synthesis of the complete mRNA, and the like can be preferably used.

The homologous recombinant cell can be obtained, for example, by introducing the above-mentioned targeting vector into target cells.

For example, when the targeting vector for gene deletion is designed to disrupt the function of an exon or a promoter of an endogenous gene by inserting other DNA fragment into the exon region or the promoter region, the vector can have, for example, the following constitution.

First, since other DNA fragment is inserted into the exon or promoter portion of the endogenous gene by homologous recombination, the targeting vector for gene deletion needs to contain sequences homologous to the target site (5' arm and 3' arm), at the 5' upstream and the 3' downstream of other DNA fragment.

While such other DNA fragment to be inserted is not particularly limited, by using a drug resistance gene or a reporter gene, target cells in which the targeting vector for gene deletion has been integrated into the chromosome can be selected using drug resistance or reporter activity as an index. Examples of the drug resistance gene include, but are not limited to, neomycin phosphotransferase II (nptII) gene, hygromycin phosphotransferase (hpt) gene, and the like, and examples of the reporter gene include, but are not limited to, β-galactosidase (lacZ) gene, chloramphenicol acetyltransferase (cat) gene, and the like.

The drug resistance or reporter gene is preferably under control of any promoter that can function in the target cell. For example, virus promoters such as SV40-derived initial promoter, cytomegalovirus (CMV) long terminal repeat (LTR), Rous sarcoma virus (RSV) LTR, mouse leukemia virus (MoMuLV) LTR, adenovirus (AdV)-derived initial promoter, and the like, as well as β-actin gene promoter, PGK gene promoter, transferrin gene promoter, and the like can be mentioned. However, when the drug resistance or reporter gene is inserted into an endogenous gene such that it is placed under the control of the endogenous promoter of HLA class I gene, a promoter controlling transcription of the gene is not necessary in the targeting vector for gene deletion.

In addition, the targeting vector for gene deletion preferably has a sequence (polyadenylation (poly A) signal, also called terminator) that terminates transcription of mRNA from the drug resistance or reporter gene at the downstream of the gene. For example, terminator sequences derived from viral genes or derived from various mammalian or avian genes can be used. Preferably, an SV40-derived terminator or the like is used.

Generally, gene recombination in cells is largely non-homologous, and the introduced DNA is randomly inserted into any location in the chromosome. Therefore, it is not possible to efficiently select only the clones with homologous recombination at the target site by selection (positive selection) such as detection of drug resistance or reporter gene expression, and confirmation of the integration site by Southern hybridization method or PCR method is necessary for all selected clones. Therefore, for example, if the herpes simplex virus-derived thymidine kinase (HSV-tk) gene that confers ganciclovir sensitivity is ligated outside the sequence homologous to the target site of the targeting vector for gene deletion, cells randomly inserted with the vector has the HSV-tk gene and cannot grow in a ganciclovir-containing medium, but cells targeted to the endogenous gene locus by homologous recombination are free of the HSV-tk gene and thus ganciclovir resistant, and are selected (negative selection). Alternatively, if, for example, a diphtheria toxin gene is ligated instead of the HSV-tk gene, cells randomly inserted with the vector are killed by the toxin produced by themselves, and homologous recombinants can also be selected in the absence of a drug.

For introduction of the targeting vector for gene deletion into the target cell, any of calcium phosphate coprecipitation method, electroporation method, lipofection method, retrovirus infection method, agglutination method, microinjection method, particle gun method, DEAE-dextran method, and the like can be used. As mentioned above, gene recombination in cells is mostly non-homologous, and the frequency of obtaining homologous recombinants is low. Therefore, the electroporation method is generally selected because it can easily process a large number of cells. For electroporation, the conditions generally used for gene transfer into animal cells can be used as they are. For example, the method can be performed by treating target cells in logarithmic growth phase with trypsin to disperse them into single cells, then suspending them in a medium at 10⁶ to 10⁸ cells/ml, transferring same into a cuvette, adding 10 to 100 µg of the targeting vector for gene deletion, and applying an electric pulse at 200 to 600 V/cm.

The target cell with the targeting vector for gene deletion integrated therein can also be detected by screening chromosomal DNAs isolated and extracted from colonies obtained by culturing single cells, by Southern hybridization or PCR method. When drug-resistant genes or reporter genes are used as other DNA fragments, transformants can be selected at the cell stage by using the expression thereof as an index. For example, when a vector containing the nptII gene is used as a positive selection marker gene, target cells after gene transfer treatment are cultured in a medium containing a neomycin antibiotic such as G418 and the like, and the emerged resistant colonies are selected as candidate transformants. When a vector containing the HSV-tk gene is used as a marker gene for negative selection, the cells are cultured in a medium containing ganciclovir, and the emerged resistant colonies are selected as candidate homologous recombinant cells. After transferring each of the obtained colonies to a culture plate and repeating trypsin treatment and medium exchange, some of the colonies are left for culture, and the rest are subjected to PCR or Southern hybridization to confirm the presence of the introduced DNA.

In addition, when a virus is used as a targeting vector for gene deletion, a method for infecting target cells with a virus containing a DNA containing a positive selection marker gene inserted between the 5' arm and the 3' arm and a negative selection marker gene outside the arm can be mentioned. When, for example, retrovirus or lentivirus is used, the cells are plated on a suitable culture vessel such as dish and the like, the virus vector is added to the culture medium (polybrene may be co-present when desired) and, after culturing for 1-2 days, a selection agent is added as described above, culture is continued, and cells that have integrated the vector are selected.

Another preferable embodiment of deleting the endogenous gene is, for example, CRISPR-Cas9 (Clustered Regularly Interspaced Short Palindromic Repeats CRISPR-Associated proteins 9) system. According to the CRISPR-Cas9 system, gene mutation can be introduced by cleaving any region on the genomic DNA by using Cas9, which is a genomic DNA cleavage enzyme, and sgRNA, which is an RNA molecule that recognizes a target site on the genome. A base in/del generated in the in vivo repair process associated with the cleavage of genomic DNA causes a frameshift in the DNA encoding amino acids, and the target endogenous gene is deleted.

Cas9 functions as an endonuclease that recognizes protospacer adjacent motif (PAM) sequence in DNA and cleaves same at the upstream thereof. Cas9 contains two functional domains with endonuclease activity and can cleave double-stranded DNA to have blunt ends. Specifically, for example, Cas9 forms a complex with a single-stranded nucleic acid (sgRNA) containing a base sequence (CRISPR-RNA (crRNA)) that specifically binds to an endogenous gene, and generates a double-strand break (DSB) at the 5'-side of PAM in the endogenous gene. Therefore, in the present invention, Cas9 means a protein that forms a complex with guide RNA and has double-stranded DNA cleavage activity.

Examples of Cas9 include, but are not limited to, SpCas9 derived from Streptococcus pyogenes, StCas9 derived from Streptococcus thermophilus, NmCas9 derived from Neisseria meningitidis, and the like.

Cas9 may also be a mutant Cas9. Mutant Cas9 is not particularly limited as long as it is a protein that maintains the ability to form a complex with guide RNA and has a mutation in which one of the two functional domains having endonuclease activity contained in Cas9 is inactivated. Examples of such mutant Cas9 include Cas9 with one mutation selected from the group consisting of a mutation in which the 10th aspartic acid of Cas9 is substituted with alanine (D10A mutation), a mutation in which the 840th histidine is substituted with alanine (H840A mutation), and/or a mutation in which the 863rd asparagine is substituted with alanine (N863A mutation).

The crRNA is not particularly limited as long as it has a base sequence complementary to the endogenous gene and adjacent to the 5' side of PAM in the endogenous gene. The length of the base sequence of crRNA is not particularly limited as long as the specificity to the endogenous gene can be secured. It is generally 10 to 30 bases long, preferably 15 to 25 bases long, more preferably 20 bases long.

PAM varies depending on the type of Cas9. For example, it is NGG (N is A, G, T, or C, same below) when Cas9 (SpCas9) derived from Streptococcus pyogenes is used, NNAGAAW when Cas9 derived from Streptococcus thermophilus (StCas9) is used, or NNNNGATT when Cas9 derived from Neisseria meningitidis (NmCas9) is used.

A single-stranded nucleic acid (sgRNA) containing a base sequence that specifically binds to an endogenous gene may further contain a base sequence necessary for recruiting Cas9 (transactivating RNA (tracrRNA)) . A known base sequence can be used for the base sequence of tracrRNA. The tracrRNA may be directly ligated to the 3'-end of crRNA, or may be ligated via a spacer sequence.

Alternatively, crRNA and tracrRNA may be associated via a complementary binding (that is, they may form a double-stranded nucleic acid) . Even in the case of gene editing using such a double-stranded nucleic acid, the introduction method into target cells described below is the same as that in the case of introducing a single-stranded nucleic acid.

Introduction of Cas9 and sgRNA into target cells can be performed according to known means. For example, Cas9 and sgRNA can be introduced into a target cell by inserting a nucleic acid sequence encoding Cas9 and a nucleic acid sequence that transcribes sgRNA into a suitable expression vector, and then introducing the expression vector into the target cell.

Nucleic acid sequences encoding Cas9 include genomic DNA, synthetic DNA, and the like. Genomic DNA encoding Cas9 can be directly amplified by Polymerase Chain Reaction (hereinafter abbreviated as "PCR method") using a primer set complementary to the cas9 gene, and using a genomic DNA fraction prepared from the aforementioned microorganism as a template. In addition, nucleic acid sequences that transcribe sgRNA can also be produced by DNA synthesis and PCR method.

An expression vector containing a nucleic acid sequence encoding Cas9 and a nucleic acid sequence that transcribes sgRNA can be produced, for example, by ligating a nucleic acid sequence fragment encoding Cas9 and a nucleic acid sequence fragment that transcribes sgRNA downstream of the promoter in a suitable expression vector.

As the expression vector, animal cell expression plasmids (e.g., pA1-11, pXT1, pRc/CMV, pRc/RSV, pcDNAI/Neo); animal virus vectors such as retrovirus, lentivirus, adenovirus, adeno-associated virus, and the like; and the like are used.

As the promoter, any promoter may be used as long as it is suitable for the host used for gene expression.

For example, SRα promoter, SV40 promoter, LTR promoter, CMV (cytomegalovirus) promoter, RSV (Rous sarcoma virus) promoter, MoMuLV (Moloney mouse leukemia virus) LTR, HSV-TK (simple herpes virus thymidine kinase) promoter, and the like are used. Among these, CMV promoter, SRα promoter, and the like are preferred.

As the expression vector in addition to the above, expression vectors optionally containing enhancers, polyA addition signals, selection markers, SV40 replication origins (hereinafter sometimes abbreviated as SV40 ori), and the like can be used when desired. Examples of the selection marker include dihydrofolate reductase gene (hereinafter sometimes to be abbreviated as dhfr, methotrexate (MTX) resistance), neomycin resistance gene (hereinafter sometimes to be abbreviated as neor, G418 resistance), and the like.

By introducing the above-mentioned expression vector containing a nucleic acid sequence encoding Cas9 and a nucleic acid sequence that transcribes sgRNA into target cells and culturing them, Cas9 and sgRNA form a complex in the target cells and the endogenous gene to be the target of sgRNA is cleaved. In the endogenous gene cleaved by Cas9, small insertions and/or deletions (in/dels) are introduced during repair of DSB by the non-homologous end joining (NHEJ) pathway, resulting in a frameshift and site-specific mutations or disruptions of the endogenous gene.

As Cas9, the Cas9 protein itself may be used without using an expression vector exemplified above. The endogenous gene to be the target of sgRNA can also be cleaved by combining Cas9 protein with sgRNA to form a complex and introducing the complex into the target cells.

As described above, the hypoimmunogenic human cell of the present invention lacks endogenous genes encoding α chain of HLA class Ia.

HLA class II is a transmembrane protein found only in antigen presenting cells such as macrophages, dendritic cells and B cells.

HLA class II presents, on the cell surface, peptide antigens derived from extracellular proteins, including proteins of extracellular pathogens taken up by immune cells through phagocytosis and the like. The peptide antigens presented by HLA class II interact with the TCR of CD4-positive helper T cells and activate CD4-positive helper T cells. The activated T cells recognize and activate B cells similarly presenting peptide antigen by HLA class II, whereby events such as phagocyte mobilization, local inflammation, humoral response, CTL activation, and the like are caused. Therefore, cells deficient in HLA class II expression can avoid development of the above-mentioned events and thus are suitable as transplantation cell sources.

HLA class II is a dimer consisting of two homologous subunits α chain and β chain. Therefore, in one embodiment of the hypoimmunogenic human cell of the present invention, endogenous genes encoding the α chain and/or β chain of HLA class II are deleted so as to render the expression of HLA class II defective. As the endogenous genes encoding HLA class II deficient in the hypoimmunogenic human cells of the present invention, at least one endogenous gene selected from the group consisting of endogenous genes encoding α chain and/or β chain of HLA-DP, endogenous genes encoding α chain and/or β chain of HLA-DQ, endogenous genes encoding α chain and/or β chain of HLA-DR, endogenous genes encoding α chain and/or β chain of HLA-DM, and endogenous genes encoding α chain and/or β chain of HLA-DO, preferably endogenous genes encoding α chain and/or β chain of HLA-DP, endogenous genes encoding α chain and/or β chain of HLA-DQ, endogenous genes encoding α chain and/or β chain of HLA-DR, endogenous genes encoding α chain and/or β chain of HLA-DM, and endogenous genes encoding α chain and/or β chain of HLA-DO can be mentioned.

In addition, the expression of each gene of HLA class II is regulated by an expression regulator therefor. The expression control mechanism is not particularly limited, and the expression (e.g., transcription) may be regulated by binding directly to the target gene, or the expression (e.g., transcription) may be controlled by binding indirectly to the target gene. For example, RFXANK regulates the transcription of target HLA class II genes by directly binding to DNA. Therefore, in the hypoimmunogenic human cell of the present invention, an endogenous gene encoding the HLA class II expression regulator is deleted as another embodiment in which the expression of HLA class II is defective. Examples of the endogenous gene encoding the HLA class II expression regulator include an endogenous gene encoding RFXANK, an endogenous gene encoding RFX5, an endogenous gene encoding RFXAP, and an endogenous gene encoding CIITA, and an endogenous gene encoding RFXANK is preferred.

A specific means of deleting endogenous genes encoding HLA class II or an expression regulator thereof may be the same as the above-mentioned means of deleting endogenous genes encoding HLA class Ia.

The hypoimmunogenic human cell of the present invention includes exogenous genes encoding α chain of HLA class Ib.

HLA class Ib has various functions. Examples of such function include presentation of specific antigens, regulation of NK cell activity, and role as Fc receptor. In addition, the function of suppressing the activation of antigen-presenting cell groups such as B cells, macrophages, monocytes, and dendritic cells can also be mentioned.

As mentioned above, endogenous genes encoding α chain of HLA class Ia and endogenous genes encoding HLA class II or expression regulator are deleted in the hypoimmunogenic human cell of the present invention, but an endogenous gene encoding β2 microglobulin is not deleted. That is, it was considered that endogenous β2 microglobulin is not defective in expression, and naturally, endogenous HLA class Ib is also not defective in expression. However, it was clarified that the expression of intact HLA class Ib (HLA-E) was also lost in the production process of the above-mentioned cells. Cells that do not express HLA class Ib (HLA-E) are inconvenient for use as a cell source for transplantation because they are rejected by NK cells. Therefore, to complement the expression of endogenous HLA class Ib, the hypoimmunogenic human cell of the present invention contains exogenous genes encoding α chain of HLA class Ib.

As the exogenous genes encoding α chain of HLA class Ib that is introduced into the hypoimmunogenic human cell of the present invention, at least one gene selected from the group consisting of exogenous genes encoding α chain of HLA-E, exogenous genes encoding α chain of HLA-F, and exogenous genes encoding α chain of HLA-G, preferably exogenous genes encoding α chain of HLA-E and/or exogenous genes encoding α chain of HLA-G can be mentioned. Furthermore, since the signal peptide of the α chain of HLA-G is important for the expression of HLA-E on the membrane surface, as the exogenous genes encoding α chain of HLA class Ib to be introduced into the hypoimmunogenic human cell of the present invention, exogenous genes encoding the α chain of HLA-E and exogenous genes encoding the α chain of HLA-G are most preferred. When the hypoimmunogenic human cell of the present invention is used as a cell source for transplantation, the exogenous genes encoding α chain of HLA class Ib to be introduced are preferably the same as the genes encoding the α chain of allele of the recipient HLA class Ib.

In the present specification, the introduction of an exogenous gene means that the exogenous gene can be expressed in the target cell by introducing the exogenous gene into the target site of the genome.

As a specific means of introducing an exogenous gene, the DNA of the exogenous gene is isolated according to a conventional method, and a DNA fragment of the exogenous gene is inserted into, for example, the target site of the target cell, thereby consequently constructing a DNA strand having a DNA sequence such that the exogenous gene is expressed in the target cell (hereinafter to be abbreviated as targeting vector for gene transfer), and a method of integrating the DNA strand into the target site of the target cell by homologous recombination may be preferably used. According to the homologous recombination method, the gene insertion site is fixed. Therefore, if there is no random integration, it is expected that the difference in the expression level between clones will be small and an influence on other genes will be less.

The homologous recombinant cell can be obtained, for example, by introducing the above-mentioned targeting vector into target cells.

For example, when the targeting vector for gene transfer is designed to express the exogenous gene in the target cell by inserting a DNA fragment of the exogenous gene into the target site, the vector can have, for example, the following constitution.

First, for insertion of the DNA fragment of the exogenous gene into the target site by homologous recombination, the targeting vector for gene transfer must contain sequences (5' arm and 3' arm) respectively homologous to the target site in the 5' upstream and 3' downstream of the DNA fragment of the exogenous gene.

In order to select target cells in which the targeting vector for gene transfer has been integrated into the chromosome, the targeting vector for gene transfer preferably contains a drug resistance gene or a reporter gene in addition to the exogenous gene to be inserted. Here, the drug resistance gene and reporter gene may be the same as those used in the targeting vector for gene deletion.

The drug resistance or reporter gene is preferably under the control of any promoter that can function in the target cell. The promoter here may be the same as that used for the targeting vector for gene deletion.

The targeting vector for gene transfer preferably has a poly A signal at the downstream of the drug resistance or reporter gene, and the signal may be the same as that used for the targeting vector for gene deletion.

It is preferable to ligate the HSV-tk gene or diphtheria toxin gene outside the sequence homologous to the target site of the targeting vector for gene transfer, because the cells targeted to the target site by homologous recombination can be selected.

The same method as that used for the targeting vector for gene deletion may be used to introduce the targeting vector for gene transfer into the target cell.

The homologous recombinant cell in which the targeting vector for gene transfer has been integrated may be selected by the same method as the method for selecting homologous recombinant cell into which the targeting vector for gene deletion has been integrated.

In addition, when a virus is used as a targeting vector for gene transfer, a method for infecting target cells with a virus containing a DNA containing an exogenous gene and a positive selection marker gene inserted between the 5' arm and the 3' arm and a negative selection marker gene outside the arm can be mentioned. The virus, the method for infecting cells, the method for selecting cells into which the vector has been integrated, and the like may be the same as the viruses and methods used for the targeting vector for gene deletion.

The target site of the targeting vector for gene transfer is not particularly limited as long as the exogenous gene can be expressed in the target cell. Examples of such site include safe harbor regions in the genome. Here, the safe harbor region is a site where the phenotype does not change even if an exogenous gene is integrated, and is selected as a target site for integrating the exogenous gene into cells used as pharmaceuticals. In the hypoimmunogenic human cell, such safe harbor region in the exogenous gene includes AAVS1 (Adeno-associated virus integration site 1) region, CCR5 (C-C chemokine receptor 5) region, ROSA26 region, and the like. Introduction of an exogenous gene into a site other than the safe harbor region is not preferable because an unexpected phenotype may be derived due to disruption of the gene at the introduced site, or the expression of the introduced exogenous gene may be suppressed. When an exogenous gene is introduced into the safe harbor region, the integration position of the exogenous gene is fixed, and it is expected that the difference in the expression level of the exogenous gene between the obtained homologous recombinants and the influence on other genes will be small.

Another preferred embodiment for introducing an exogenous gene is the PiggyBac method. In the PiggyBac method, a transposon vector into which a DNA fragment containing an exogenous gene has been integrated and a transposase expression vector that expresses transposase are used. The genes and the like contained in the transposon vector and the transposase expression vector may be present separately in the above-mentioned separate vectors, or may be contained in a single vector. The transposon vector and the transposase expression vector may have, for example, the following constitutions.

In order to excise a DNA fragment containing an exogenous gene from the transposon vector by transposase, the transposon vector contains a terminal inverted repeat sequence (Terminal Inverted Repeat) at the 5' upstream and 3' downstream of the DNA fragment containing the exogenous gene. Transposase recognizes a terminal inverted repeat sequence contained in a transposon vector, and excises a DNA fragment containing an exogenous gene sandwiched between the terminal inverted repeat sequences from the transposon vector.

In order to select target cells in which the exogenous gene has been integrated into the target site, the transposon vector preferably contains a drug resistance gene or a reporter gene in addition to the DNA fragment containing the exogenous gene. Here, the drug resistance gene and reporter gene may be the same as those used in the targeting vector for gene transfer.

The drug resistance and reporter genes are preferably under the control of any promoter that can function in the target cell. The promoter here may be the same as that used for the targeting vector for gene transfer.

The transposon vector preferably has a poly A signal at the downstream of the drug resistance or reporter gene, and the signal may be the same as that used for the targeting vector for gene transfer.

The transposase expression vector may contain drug resistance gene, reporter gene, promoter, poly A signal, and the like in addition to a gene encoding transposase. The drug resistance gene, reporter gene, promoter, and poly A signal may be the same as those contained in the transposon vector.

The same method as that used for the targeting vector for gene transfer may be used for the introduction of the transposon vector and the transposase expression vector into the target cell.

The cell in which the exogenous gene has been integrated into the target site may be selected by the same method as the method for selecting homologous recombinant cell into which the targeting vector for gene transfer has been integrated.

Using the transposon vector and the transposase expression vector into which the DNA fragment of the exogenous gene introduced as described above has been integrated, the DNA fragment of the exogenous gene can be integrated into the transposase target sequence TTAA in the genome of the target cell. In this method, since the target sequence is TTAA unlike the homologous recombination method using the above-mentioned targeting vector for gene transfer, the site into which the exogenous gene is integrated cannot be limited. However, it is also possible to remove the exogenous gene integrated in the genome, without leaving a trace by expressing the transposase thereafter.

The hypoimmunogenic human cell of the present invention also contains an exogenous gene encoding human PD-L1 and an exogenous gene encoding human PD-L2.

PD-L1 and PD-L2 are transmembrane type proteins belonging to the immunoglobulin superfamily and are known as immune tolerance factors. Specifically, PD-L1 and PD-L2 play an important role as checkpoints in autoimmune tolerance, excess immunity, and inflammatory responses, since they suppressively control peripheral immunoactivity. Cells that constitutively express PD-L1 and PD-L2 can suppress proliferation and injury function of T cell and avoid immune responses. Therefore, the hypoimmunogenic human cell of the present invention contains an exogenous gene encoding human PD-L1 and an exogenous gene encoding human PD-L2.

A specific means of introducing an exogenous gene encoding PD-L1 and an exogenous gene encoding PD-L2 may be the same as the above-mentioned means of introducing the above-mentioned exogenous genes encoding α chain of HLA class Ib.

The hypoimmunogenic human cell of the present invention can be obtained as described above. As described above, the hypoimmunogenic human cell of the present invention can avoid immune responses. In addition, the hypoimmunogenic human cell of the present invention maintains characteristics of parental cell. For example, when a cell that deletes the above-mentioned endogenous genes (1) and (2) and introduces the above-mentioned exogenous genes (3), (4), and (5) is the human pluripotent stem cell, the obtained hypoimmunogenic human cell of the present invention maintains expression of undifferentiated markers and has similar all gene expression patterns, like the original human pluripotent stem cells. Furthermore, the hypoimmunogenic human cell of the present invention maintains the differentiation potential into various cells, like the original human pluripotent stem cells.

The inventors next analyzed the hypoimmunogenic human cell of the invention into which an exogenous gene encoding β2 microglobulin was further introduced.

In the hypoimmunogenic human cell of the present invention, an endogenous gene encoding β2 microglobulin is not originally deficient in expression. Initially, therefore, it was considered that introduction of an exogenous gene encoding β2 microglobulin would not significantly affect the expression of HLA class Ib on the cell surface in the hypoimmunogenic human cell of the present invention. In fact, there are reports on the example of cells with only exogenous genes encoding α chain of HLA class Ib introduced thereinto, and an example in which the α-chain and β-chain (β2-microglobulin) of HLA class Ib are linked and expressed as a single molecule in cells in which a gene encoding β2-microglobulin is deleted to render the expression of HLA class I deficient. However, the idea of further introducing an exogenous gene encoding β2-microglobulin into a cell having endogenous β2-microglobulin was never conceived, and no such example has been reported. Unexpectedly, however, it was found that the numbers of HLA class Ib α-chain and β2-microglobulin molecules on the cell surface increased in the hypoimmunogenic human cell of the present invention into which an exogenous gene encoding β2 microglobulin was additionally introduced.

Therefore, in order to increase the hypoimmunogenicity of the hypoimmunogenic human cell of the present invention by increasing the number of HLA class Ib molecules on the cell surface, the hypoimmunogenic human cell of the present invention may further contain an exogenous gene encoding human β2 microglobulin. Such hypoimmunogenic human cell further containing an exogenous gene encoding exogenous human β2 microglobulin has not been reported to date, and excessive trial and error is required for those skilled in the art to arrive at such constitution.

From the above, it can be said that the hypoimmunogenic human cell of the present invention is a completely new hypoimmunogenic cell that cannot be achieved from conventional ideas. Therefore, the hypoimmunogenic human cell of the present invention further introduced with an exogenous gene encoding β2-microglobulin can be expected to exhibit higher hypoimmunogenicity than before introduction, by increasing the number of HLA class I molecules on the cell surface.

The β2 microglobulin is a common component that associates with the α chains of HLA class I (HLA class Ia and HLA class Ib). A specific means of introducing an exogenous gene encoding β2 microglobulin may be the same as the above-mentioned means of introducing the above-mentioned exogenous genes encoding α chain of HLA class Ib.

The hypoimmunogenic human cell of the present invention may also contain a suicide gene. Particularly, when a cell that deletes the above-mentioned endogenous genes (1) and (2) and introduces the above-mentioned exogenous genes (3), (4), and (5) is the human pluripotent stem cell, in order to avoid the risk of tumorigenesis and the like that the obtained hypoimmunogenic human cell of the present invention has, it is desirable to introduce a suicide gene. This makes it possible to remove only the hypoimmunogenic human cell of the present invention when it causes undesirable side effects such as canceration after transplantation and the like.

As the suicide gene to be introduced into the hypoimmunogenic human cell of the present invention, for example, HSV-tk gene and variants thereof (e.g., HSV-TK, HSV-TK39, and the like) and iCaspase9 (e.g., AP1903-binding, Rapamycin-binding, and the like) can be mentioned, though not particularly limited thereto. A specific means of introducing a suicide gene may be the same as the above-mentioned means of introducing the above-mentioned exogenous genes encoding α chain of HLA class Ib. However, expression of the suicide gene introduced into the hypoimmunogenic human cell of the present invention is optionally manipulated. Therefore, in a targeting vector for suicide gene transfer for introducing the suicide gene, the suicide gene is linked to a conditional promoter. As the conditional promoter, a promoter containing Tet operator DNA sequence (tetO) can be mentioned. The promoter containing Tet operator DNA sequence (tetO) is driven by a complex of reverse tetracycline-controlled transactivator (rtTA) and doxycycline (Dox).

The present invention also provides a method for producing a hypoimmunogenic human cell, containing the following steps (i) to (v) (hereinafter the production method of the hypoimmunogenic human cell of the present invention):
(i) a step of deleting an endogenous gene encoding an α chain of HLA class Ia of a human parental cell,
(ii) a step of deleting an endogenous gene encoding HLA class II or an expression regulator thereof from the human parental cell,
(iii) a step of introducing an exogenous gene encoding an α chain of HLA class Ib into the human parental cell,
(iv) a step of introducing an exogenous gene encoding human PD-L1 into the human parental cell, and
(v) a step of introducing an exogenous gene encoding human PD-L2 into the human parental cell.

In the production method of the hypoimmunogenic human cell of the present invention, the human parental cells used in each of the steps (i) to (v) may be the same as the cells in the production of the hypoimmunogenic human cell of the present invention in which the endogenous genes of (1) and (2) can be deleted and the exogenous genes of (3), (4), and (5) can be introduced.

In the production method of the hypoimmunogenic human cell of the present invention, the endogenous genes to be deleted and the exogenous genes to be introduced in each of the steps (i) to (v) may be the same as the endogenous genes to be deleted and the exogenous genes to be introduced in the hypoimmunogenic human cell of the present invention. In the production method of the hypoimmunogenic human cell of the present invention, moreover, the specific means of deleting and the specific means of introducing the exogenous genes in each of the steps (i) to (v) may be the same as the specific means of deleting and the specific means of introducing the exogenous genes in the hypoimmunogenic human cell of the present invention. In the production method of the hypoimmunogenic human cell of the present invention, the target site into which the exogenous genes are introduced in each of the steps (iii) to (v) may be the same as the target site into which the exogenous genes are introduced in the hypoimmunogenic human cell of the present invention. In the production method of the hypoimmunogenic human cell of the present invention, the respective steps (i) to (v) may be performed in any order as long as the hypoimmunogenic human cell of the present invention can be obtained.

The hypoimmunogenic human cell obtained as described above avoids immune responses and maintains characteristics of parental cell, like the hypoimmunogenic human cell of the present invention. For example, when the human parental cell is a human pluripotent stem cell, the obtained hypoimmunogenic human cell maintains the expression level of undifferentiated markers, has very similar all gene expression patterns, and maintains differentiation potential into various cells, like the parental human pluripotent stem cell.

The production method of the hypoimmunogenic human cell of the present invention may further contain (vi) a step of introducing an exogenous gene encoding human β2 microglobulin into the human parental cell. In the production method of the hypoimmunogenic human cell of the present invention, the human parental cell used in step (vi), an exogenous gene encoding the introduced human β2 microglobulin, the specific means of introducing the exogenous genes, the target site into which the exogenous genes are introduced, and the like may be the same as those described in the production of the hypoimmunogenic human cell of the present invention.

The hypoimmunogenic human cell of the present invention obtained as described above by further introducing an exogenous gene encoding β2 microglobulin is expected to achieve higher hypoimmunogenicity by increasing the number of HLA class I molecules on the cell surface.

The production method of the hypoimmunogenic human cell of the present invention may further contain (vii) a step of introducing a suicide gene into the human parental cell. In the production method of the hypoimmunogenic human cell of the present invention, the human parental cell used in step (vii), the suicide gene to be introduced, the specific means of introducing the suicide gene, the target site into which the suicide gene is introduced, and the like may be the same as those described in the production of the hypoimmunogenic human cell of the present invention.

The hypoimmunogenic human cell of the present invention obtained as described above by further introducing a suicide gene permits removal of only the hypoimmunogenic human cell of the present invention when it causes undesirable side effects such as canceration after transplantation and the like.

The present invention also provides a medicament containing the hypoimmunogenic human cell of the present invention (hereinafter the medicament of the present invention). The hypoimmunogenic human cell of the present invention can avoid immune responses, and thus can be used as a cell source for transplantation. Therefore, a medicament containing the hypoimmunogenic human cell of the present invention can be used as a medicament for regenerative therapy.

The medicament of the present invention is preferably used by parenteral administration to a subject. Examples of the parenteral administration method include intravenous, intraarterial, intramuscular, intraperitoneal, and subcutaneous administration methods and the like. The dosage is appropriately selected according to the condition, body weight, age, and the like of the subject. Generally, the number of cells per dose is 1×10⁶ to 1×10¹⁰ in the case of a subject with body weight 60 kg. Moreover, it may be administered once or in multiple doses. The medicament of the present invention can be in a known form suitable for parenteral administration, for example, injection or infusion. The medicament of the present invention may contain physiological saline, phosphate-buffered saline (PBS), medium, and the like in order to maintain cells stably. In addition, pharmaceutically acceptable carriers (e.g., human serum albumin), preservatives, and the like may be added to the medicament for the purpose of stabilization thereof.

### [Example]

The present disclosure is explained in more detail in the following by referring to Examples. However, they are examples and the present disclosure is not limited to these Examples.

### [Example 1] Production of HLA class Ia-deficient iPS cell

In order to avoid immunorejection of T cells caused by HLA mismatch, the α-chain genes of HLA-A, HLA-B, and HLA-C belonging to HLA class Ia were deleted. The CRISPR-Cas9 method was used for the gene deletion.

The following crRNA sequences were used as guide RNAs (gRNAs) for each α chain gene of HLA class Ia. The underline shows PAM sequence.
#HLA-A: ACAGCGACGCCGCGAGCCAGAGG(SEQ ID NO: 1)
#HLA-B: CCTCCTCCGCGGGTATGACCAGG(SEQ ID NO: 2)
#HLA-C: AGCGACGCCGCGAGTCCAAGAGG(SEQ ID NO: 3)

A gRNA containing each crRNA sequence was synthesized and mixed with tracrRNA (Thermo Fisher Scientific) to produce double-stranded gRNA. The double-stranded gRNA was mixed with Cas9 protein (Alt-R S.p. HiFi Cas9 Nuclease V3, Integrated DNA Technologies) to produce a Cas9-gRNA complex. Hereinafter they are respectively referred to as "HLAA-gRNA-Cas9 complex", "HLAB-gRNA-Cas9 complex", and "HLAC-gRNA-Cas9 complex". Each complex was blended and used immediately before introduction into iPS cells.

The iPS cell clone 06E (TC-1133HKK_06E_MCB) was used as the parent strain of iPS cells. This parent strain is hereinafter referred to as "unedited iPS cell". First, the iPS cell suspension and the HLAB-gRNA-Cas9 complex were mixed, the HLAB-gRNA-Cas9 complex was introduced into unedited iPS cells using the electroporation method (Neon Transfection System, Thermo Fisher Scientific) and the cells were cultured for 5 days. After 5 days, the HLAC-gRNA-Cas9 complex was introduced and the cells were cultured for 5 more days. Thereafter, the HLAA-gRNA-Cas9 complex was introduced and the cells were further cultured for 5 more days. Single-cell cloning was performed from this gRNA-Cas9 complex-introduced iPS cell, and the gene-edited cell was isolated.

Single-cell cloning was performed by the following method. Five days after the introduction of the three types of gRNA-Cas9 complexes, the cells were seeded such that each well of a 96-well plate contained one cell as a calculated value. Twelve days after seeding on the 96-well plate, the cells of 238 clones were passaged in each well of a 24-well plate and a 96-well plate. The cells on the 96-well plate were used for screening. On the other hand, the cells on the 24-well plate were cultured continuously. One week after seeding, only the candidate clones were passaged in a 9 cm dish based on the results of screening. The cells cultured on a 9 cm dish were cryopreserved.

Screening was performed by the following method. Two days after seeding from one 96-well plate to another 96-well plate, 238 clones were immunostained with anti-HLA-A/B/C antibodies (clone W6/32) and 53 clones with drastically-decreased signal were selected. The genome was extracted from the fixed and stained cells of these 53 clones, and the presence or absence of mutation on the genome was analyzed by the In vitro Cas9 Cleavage Assay method. Specifically, each well of the 96-well plate in which the cells were cultured was first washed with a PBS solution. After removing PBS, 101 µL of a solution (TAKARA, Lysis Buffer for PCR, 9170A) in which 1 µL of Proteinase K was mixed with respect to 100 µL of Lysis Buffer was added. The cells lysed with Lysis Buffer with Proteinase K were transferred to a 0.2 mL PCR tube and reacted at 60°C for 5 min using a thermal cycler to completely lyse the cells. Then, the reaction was performed at 98°C for 2 min to inactivate the Proteiase K. The reaction was terminated by lowering the temperature to 22°C to obtain a genome extraction solution. Then, using this genome as a template, DNA fragments near the gRNA target sequences for HLA-A, HLA-B, and HLA-C were amplified by PCR. Next, this PCR fragment and gRNA-Cas9 complexes for each gene were mixed and allowed to react at 37°C for 60 min. Thereafter, agarose gel electrophoresis was performed to analyze the length of the DNA fragments. One DNA fragment is observed because the genome-edited PCR fragment is not cleaved by the gRNA-Cas9 complex. On the other hand, the PCR fragment free of genome editing is cleaved by the gRNA-Cas9 complex, resulting in two DNA fragments. A single DNA fragment is observed from a cell in which both genomes have been gene-edited and base insertion or deletion (In/Del) has occurred. When In/Del occurs in one genome and the other genome is unedited, three DNA fragments are observed. When both genomes are unedited, two DNA fragments are observed. The HLA-B locus and HLA-C locus were also analyzed in the same manner.

One-cell-derived 53 colonies showing decreased expression of HLA class I protein by immunostaining were analyzed by In vitro Cas9 Cleavage Assay. As a result, a colony with gene mutation in the both gene strands of all three gene loci of HLA-A, HLA-B, HLA-C (mutations in total 6 alleles) was not identified by observation. Therefore, 6 clones with mutations in total 5 alleles were isolated.

Then, from these 6 candidate clones, a DNA fragment containing the vicinity of the gRNA target sequence was amplified by PCR method, and the base sequence thereof was analyzed by Sanger sequencing. When two genomes have different sequences, the waveforms of the Sanger sequences overlap from the vicinity of the gRNA target sequence and become unclear. The waveform of the overlapped Sanger sequences was analyzed by online software (TIDE) (https://tide.deskgen.com/) and visual observation, the sequence of each gene chain was decoded, and the actual gene insertion/deletion was confirmed. Of these 6 clones, 2 clones (18B12 and 17E10) were confirmed to have mutations with frameshifts at 5 sites.

Gene editing results of HLA-A(-/+):HLA-B(-/-):HLA-C(-/-) cell clone 18B12 are shown below.
#HLA-A: +1nt/no mutation
#HLA-B: -10nt/-1+17nt
#HLA-C: +1nt/-13nt

The following primers were used to amplify a DNA fragment containing the vicinity of the gRNA target sequence by a PCR method.
#HLA-A, Fwd: AATCAGTGTCGTCGCGGTCG(SEQ ID NO: 4)
#HLA-A, Rev: AGTCTGTGAGTGGGCCTTCAC(SEQ ID NO: 5)
#HLA-B, Fwd: GAGACACAGATCTCCAAGACCAACA(SEQ ID NO: 6)
#HLA-B, Rev: CCTGAGAGGAAAAGTCACGGTTC(SEQ ID NO: 7)
#HLA-C, Fwd: AGGGAAACGGCCTCTGCGGA(SEQ ID NO: 8)
#HLA-C, Rev: TCTGTGCCTGGCGCTTGTAC(SEQ ID NO: 9)

The length of the PCR product when using the above-mentioned primers is as follows.
#HLA-A: 497bp
#HLA-B: 889bp
#HLA-C: 329bp

In order to introduce mutation into the HLA-A allele, in which mutation was not introduced, in the HLA-A(-/+):HLA-B(-/-):HLA-C(-/-) iPS cell clone 18B12, HLAA-gRNA-Cas9 complex was reintroduced, single-cell cloning and screening were performed in the same manner as described above, and HLA-A(-/-): HLA-B(-/-): HLA-C(-/-) iPS cells were isolated.

Four days after the introduction of the HLAA-gRNA-Cas9 complex, cells were seeded such that each well of a 96-well plate contained one cell as a calculated value. Twelve days after seeding on the 96-well plate, cells were passaged in each well of a 24-well plate and a 96-well plate.

The cells on the 96-well plate were collected the day after passage, and genomic DNA was extracted. Using the extracted genomic DNA, the presence or absence of mutation on the genome was analyzed by the In vitro Cas9 Cleavage Assay method. As a result of analyzing single-cell-derived 75 colonies (clones), one DNA fragment was observed in the HLA-A gene of 11 clones. The clones were selected as candidate clones with gene editing of both strands of the genomic DNA. Then, from these 11 candidate clones, a DNA fragment containing the vicinity of the gRNA target sequence was amplified by PCR method, and the base sequence thereof was analyzed by Sanger sequencing method. It was confirmed that a mutation accompanied by a frameshift was introduced in both chains of all isolated HLA-A genes. The HLA-A(-/-):HLA-B(-/-):HLA-C(-/-) cells isolated here are hereinafter referred to as "HLA class Ia-deficient iPS cells (HGEC-0006 cells)".

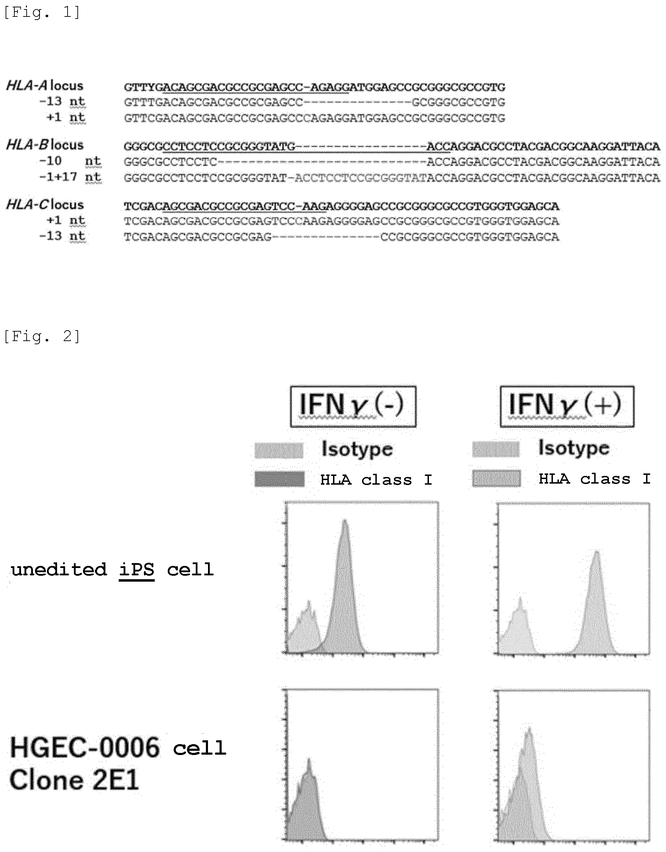

Of the 11 clones obtained, the gene editing results of HLA class Ia-deficient iPS cell clone 2E1 are shown below (Fig. 1).
#HLA-A: -13nt/+1nt
#HLA-B: -10nt/-1+17nt
#HLA-C: +1nt/-13nt

The cell surface expression level of HLA class Ia protein in HLA class Ia-deficient iPS cell clone 2E1 was analyzed using a flow cytometry method. A suspension of 200,000 iPS cells was mixed with an antibody that recognizes HLA class I (clone G46-2.6), and reacted for 30 min in a solution amount of 20 µL on ice. Thereafter, 1 mL of a 2% BSA-mixed PBS solution was added to diffuse unbound antibodies into the solution, and after centrifugation, only the pellets were recovered to collect only the cells bound to the antibodies. Using a fluorescence-labeled antibody as the antibody, the cell surface expression level of the antibody-binding protein was analyzed using flow cytometry (BD FACSVerse). As a result, a drastic decrease in the cell surface expression level of class I protein was confirmed (Fig. 2).

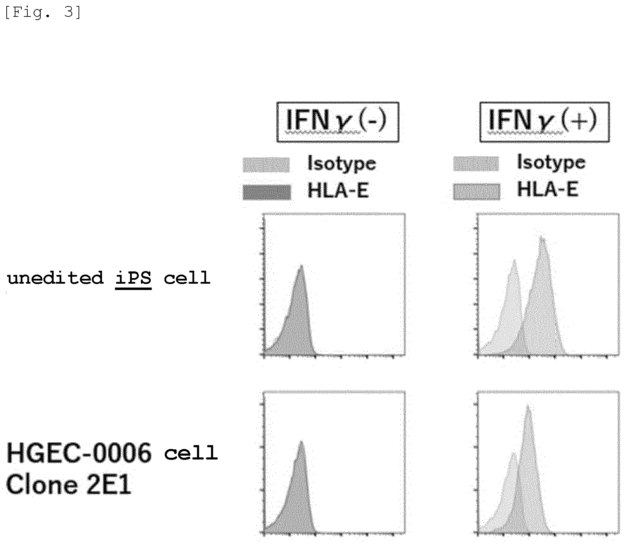

Next, for the purpose of confirming how the deficiency of HLA-A, HLA-B, and HLA-C proteins affects the expression of HLA class Ib, the cell surface expression of HLA-E in class Ia-deficient iPS cell clone 2E1 was analyzed using a flow cytometry method. An anti-HLA-E antibody (clone 3D2) was used as an antibody against HLA-E. As a result, expression of HLA-E was confirmed, but decreased to about half compared to unedited iPS cells (Fig. 3).

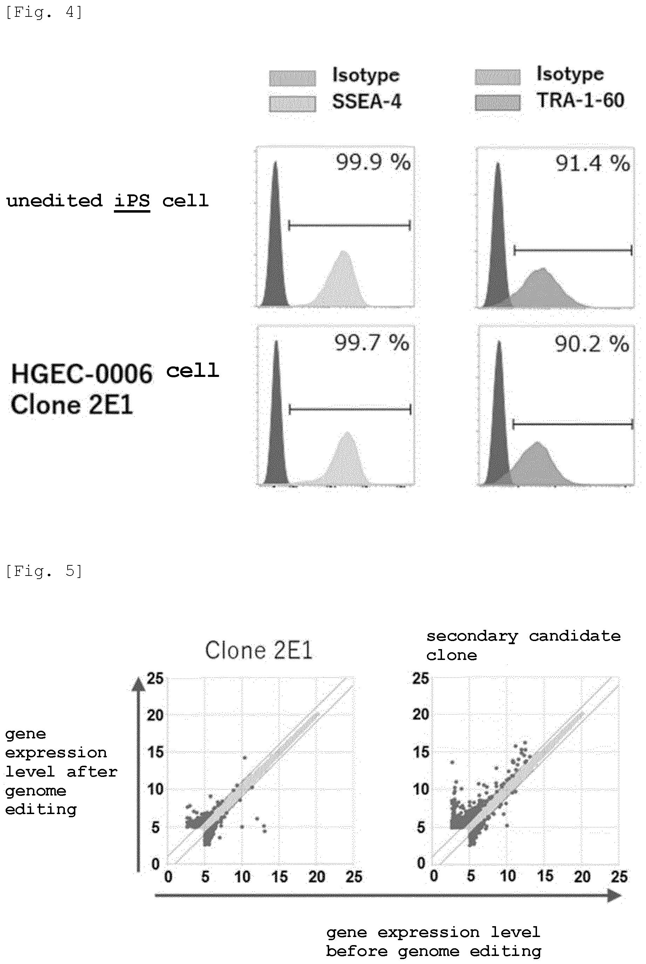

In order to confirm whether the deficiency of HLA-A, HLA-B, and HLA-C proteins caused changes in the undifferentiated state of iPS cells, expression of undifferentiated markers in HLA class Ia-deficient iPS cell clone 2E1 was analyzed by a flow cytometry method. Anti-SSEA-4 antibody (clone MC813-70) and anti-TRA-1-60 antibody (clone TRA-1-60) were used as antibodies against undifferentiated markers. As a result, it was confirmed that the expression level of the undifferentiated marker in the HLA class Ia-deficient iPS cell clone 2E1 was almost the same as that of the parent line, unedited iPS cells (Fig. 4).

In order to confirm whether the deficiency of HLA-A, HLA-B, and HLA-C proteins caused changes in the all gene expression patterns of iPS cells, transcriptome analysis (Takara Bio Inc., Agilent Array expression contract analysis) was performed. This human SurePrint G3 Human Gene Expression 8x60K v3 carries probes covering 26,083 Entrez gene RNAs and 30,606 IncRNAs. HLA class Ia-deficient iPS cell clone 2E1 and HLA class Ia-deficient iPS cell clone 2H3 (second candidate clone), and unedited iPS cells as a control group were respectively cultured in 6 cm dishes. The cells were lysed by directly adding 600 µL of Buffer RLT with 2-ME (QIAGEN) to the culture vessel, and the cells were transferred to a 1.5 mL tube. The cells were further lysed by pipetting and frozen at -80°C. As a result of Agilent Microarray expression contract analysis on this sample, it was confirmed that the RNA expression pattern in the HLA class Ia-deficient iPS cell clone 2E1 was almost the same as that of the parent strain, the unedited iPS cell clone 06E (Fig. 5). The RNA expression pattern of the second candidate clone 2H3 was also close to that of the unedited iPS cell clone 06E.

### [Example 2] Production of HLA class Ia&II-deficient iPS cell

In order to suppress immunorejection of T cells caused by HLA mismatch, RFXANK gene was further deleted from the HLA class Ia-deficient iPS cell clone 2E1 obtained in Example 1. Since the RFXANK gene is a transcription factor that controls the expression of the HLA class II gene group, deletion of the RFXANK gene is expected to drastically reduce the expression level of the HLA class II gene group. The CRISPR-Cas9 method was used for the gene deletion.

The following crRNA sequence was used as guide RNA (gRNA) for RFXANK gene. The underline shows PAM sequence.
#RFXANK: TGAGACCGTTCGCTTCCTGCTGG(SEQ ID NO: 10)

A gRNA containing crRNA sequence for RFXANK was synthesized and mixed with tracrRNA (Thermo Fisher Scientific) to produce double-stranded gRNA. The double-stranded gRNA was mixed with Cas9 protein (Alt-R S.p. HiFi Cas9 Nuclease V3, Integrated DNA Technologies) to produce a Cas9-gRNA complex. Hereinafter it is referred to as "RFXANK-gRNA-Cas9 complex". This complex was blended and used immediately before introduction into iPS cells.

RFXANK-gRNA-Cas9 complex was introduced into HLA class Ia-deficient iPS cell clone 2E1 using the electroporation method (Neon Transfection System, Thermo Fisher Scientific). single-cell cloning and screening were performed by a method similar to that in Example 1, and HLA-A(-/-):HLA-B(-/-):HLA-C(-/-):RFXANK(-/-)iPS cells were isolated.

Four days after the introduction of the RFXANK-gRNA-Cas9 complex, cells were seeded such that each well of a 96-well plate contained one cell as a calculated value. Twelve days after seeding on the 96-well plate, cells were passaged in each well of a 24-well plate and a 96-well plate. The cells on the 96-well plate were collected the day after seeding, and the genome was extracted. Using the extracted genome, the presence or absence of mutation on the genome was analyzed by the In vitro Cas9 Cleavage Assay method. On the other hand, the cells on the 24-well plate were continuously cultured, and one week after seeding, only the candidate clones were passaged in a 9 cm dish based on the screening results. The cells cultured on the 9 cm dish were cryopreserved.

Screening was performed by the following method. As a result of analyzing single-cell-derived 256 colonies (clones) using the aforementioned In vitro Cas9 Cleavage Assay method, one DNA fragment was observed in 4 clones. The clones were selected as candidate clones with gene editing of both the two genomes. Then, from these 4 candidate clones, a DNA fragment containing the vicinity of the gRNA target sequence was amplified by PCR, and the base sequence thereof was analyzed by Sanger sequencing. Of these 4 candidate clones, 2 clones were confirmed to have gene deletion mutation accompanied by frameshifts at two RFXANK gene loci. The HLA-A(-/-):HLA-B(-/-):HLA-C(-/-):RFXANK(-/-) cells isolated here are hereinafter referred to as "HLA class Ia&II-deficient iPS cells (HGEC-0009 cells)".

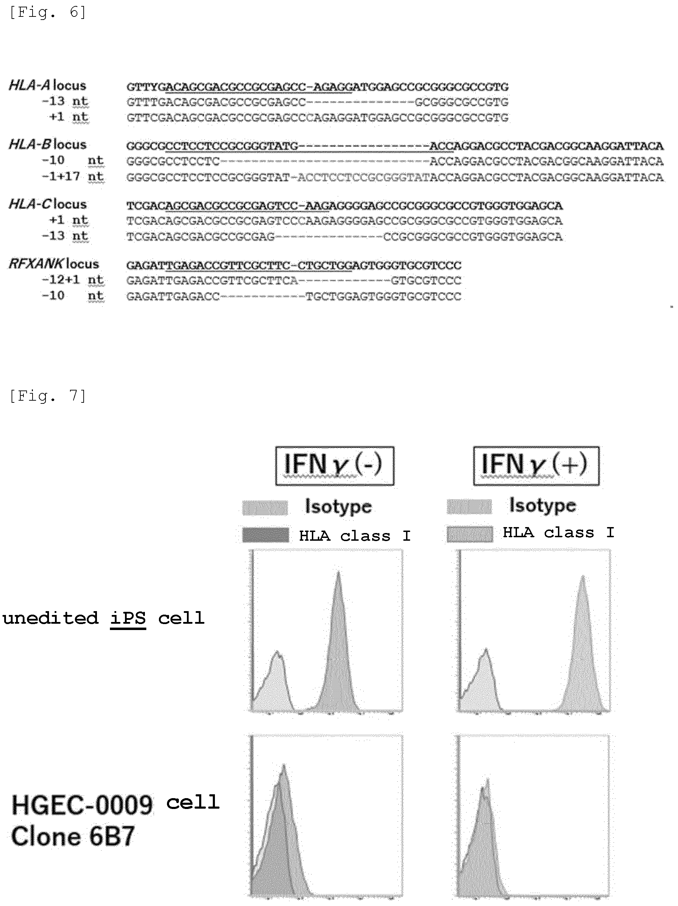

Of the 2 clones obtained, the gene editing results of HLA class Ia&II-deficient iPS cell clone 6B7 are shown below (Fig. 6).
#HLA-A: -13nt/+1nt
#HLA-B: -10nt/-17+1nt
#HLA-C: +1nt/-13nt
#RFXANK: -11nt/-10nt

The following primers were used to amplify a DNA fragment containing the vicinity of the gRNA target sequence by a PCR method.
#RFXANK, Fwd: ATACCCACTCATGACGTGACCTG(SEQ ID NO: 11)
#RFXANK, Rev: CAGCCGCATCTCAAAGACAAG(SEQ ID NO: 12)

The length of the PCR product when using the above-mentioned primers is as follows.
#RFXANK: 410bp

The cell surface expression level of HLA class Ia protein in HLA class Ia&II-deficient iPS cell clone 6B7 was analyzed using a flow cytometry method in the same manner as in Example 1. As a result, a drastic decrease in the cell surface expression level of HLA class I protein was confirmed in HLA class Ia&II-deficient iPS cell clone 6B7, like the cells obtained in Example 1 (Fig. 7) .

For the confirmation of the cell surface expression level of HLA class II protein in HLA class Ia&II-deficient iPS cell clone 6B7, the iPS cells need to be induced to differentiate into HLA class II-expressing cells. Therefore, hematopoietic cells including iPS cell-derived dendritic cell-like cells were utilized as such HLA class II-expressing cells. Differentiation from iPS cells into hematopoietic progenitor cells followed the literature (Biochem Biophys Res Commun. 2019 Jul 12; 515(1) :1-8.). In order to induce the differentiation of hematopoietic differentiated cells into hematopoietic cells including dendritic cell-like cells, iPS cell-derived hematopoietic progenitor cells were seeded on OP9 feeder cells, 100 ng/mL FLT3L, 20 ng/mL SCF, and 20 ng/mL GM-CSF were added, and the cells were cultured for 4-7 days. In order to detect the appearance of dendritic cell-like cells, antibodies that recognize dendritic cell markers, anti-CD11c antibody (clone REA618, MiltenyBiotec, 130-114-110), and anti-CD11b antibody (clone ICRF44, BD Pharmingen, 558123) were used.

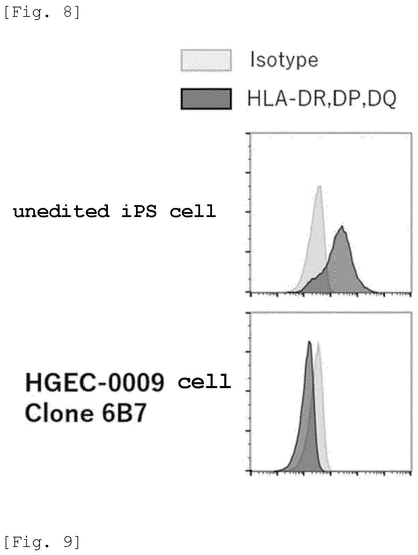

In order to confirm the decrease in the expression level of HLA class II protein due to RFXANK deficiency, iPS cells were differentiated into hematopoietic cells including dendritic cells by the above-mentioned method, and the cell surface expression levels of HLA class II proteins in HLA class Ia&II-deficient iPS cell clone 6B7-derived dendritic cell-like cells were analyzed using a flow cytometry method. As antibodies that recognize dendritic cell markers, anti-CD11c antibody (clone REA618, MiltenyBiotec, 130-114-110) and anti-CD11b antibody (clone ICRF44, BD Pharmingen, 558123) were used. As an antibody that recognizes HLA class II, anti HLA-DR/DQ/DP (clone Tu39, BD Pharmingen, 557715) was used (Fig. 8).

For the purpose of confirming the expression of HLA class Ib in the case of deletion of RFXANK in addition to the deletion of HLA-A, HLA-B, and HLA-C proteins, cell surface expression of HLA-E in HLA class Ia&II-deficient iPS cell clone 6B7 was analyzed by a flow cytometry method, as in Example 1. As an antibody that detects HLA-E, an anti-HLA-E antibody (clone 3D2) was used. As a result, expression of HLA-E could not be detected in the HLA class Ia&II-deficient iPS cell clone 6B7 (Fig. 9).

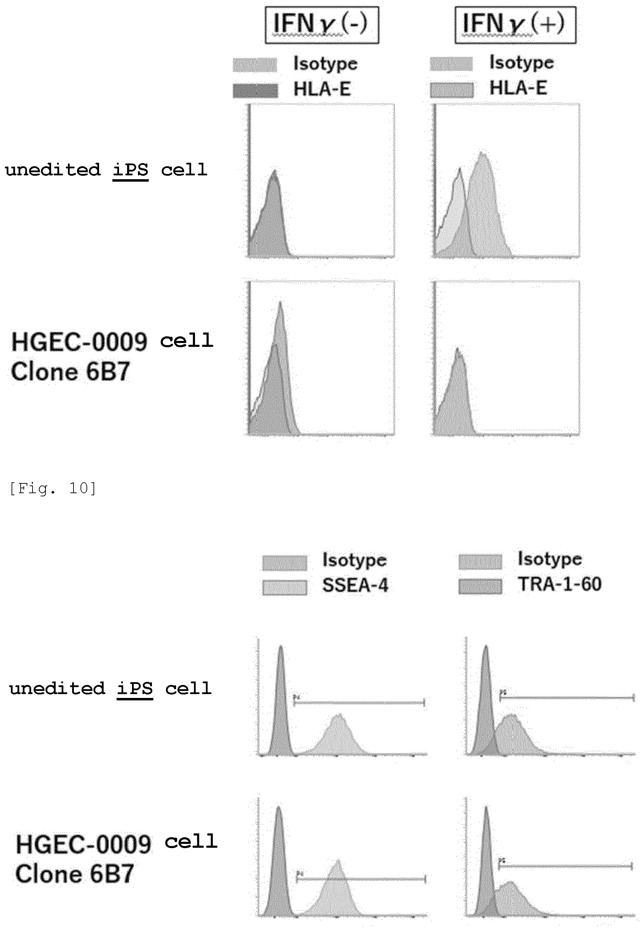

In order to confirm whether the deletion of HLA-A, HLA-B, and HLA-C proteins in addition to the deletion of RFXANK caused changes in the undifferentiated state of iPS cells, cell surface expression of undifferentiated markers in HLA class Ia&II-deficient iPS cell clone 6B7 was analyzed by a flow cytometry method as in Example 1. As a result, it was confirmed that the expression level of the undifferentiated marker in the HLA class Ia&II-deficient iPS cell clone 6B7 was almost the same as that of the parent strain, unedited iPS cell clone 06E (Fig. 10).

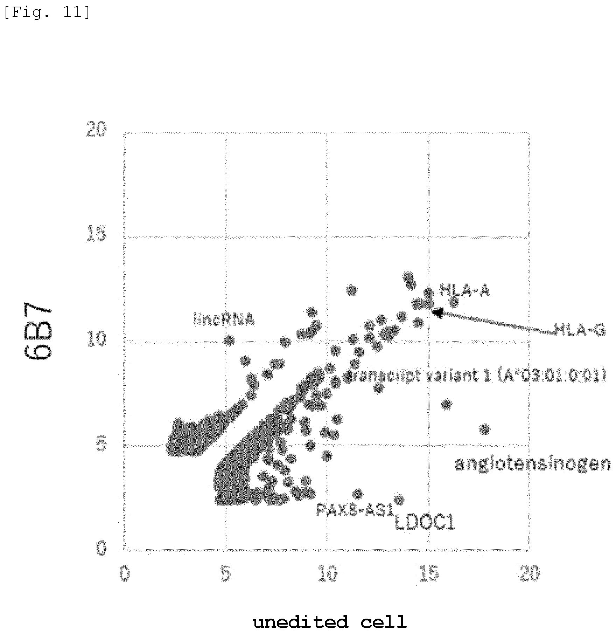

Whether the lack of RFXANK in addition to the lack of HLA-A, HLA-B, and HLA-C proteins caused changes in all gene expression patterns of iPS cells was analyzed by a method similar to that in Example 1. As a result, it was confirmed that the RNA expression pattern in the HLA class Ia&II-deficient iPS cell clone 6B7 was equivalent to that of the parent strain, unedited iPS cell clone 06E (Fig. 11).

HLA class Ia&II-deficient iPS cell clone 6B7 was confirmed to maintain differentiation potential into multiple lineages. To be specific, induction of differentiation into vascular endothelial cells, induction of differentiation into hematopoietic cells, and induction of differentiation into hepatic cells were respectively performed, and it was confirmed that the induction of differentiation was performed normally. The method of inducing differentiation into each cell is shown below.

Induction of differentiation from iPS cells into hematopoietic progenitor cells was performed according to the literature (Biochem Biophys Res Commun. 2019 Jul 12; 515(1):1-8.). As one of the evaluations of the differentiation potential into hematopoietic progenitor cells, the cell surface expression of hematopoietic progenitor cell-specific proteins was measured by a flow cytometry method. As hematopoietic progenitor cell markers, anti-CD45 antibody (clone HI30, BD Pharmingen, 563880), anti-CD43 antibody (clone 1G10, BD Pharmingen, 555475), and anti-CD34 antibody (clone 8G12, BD Pharmingen, 340441) were used.

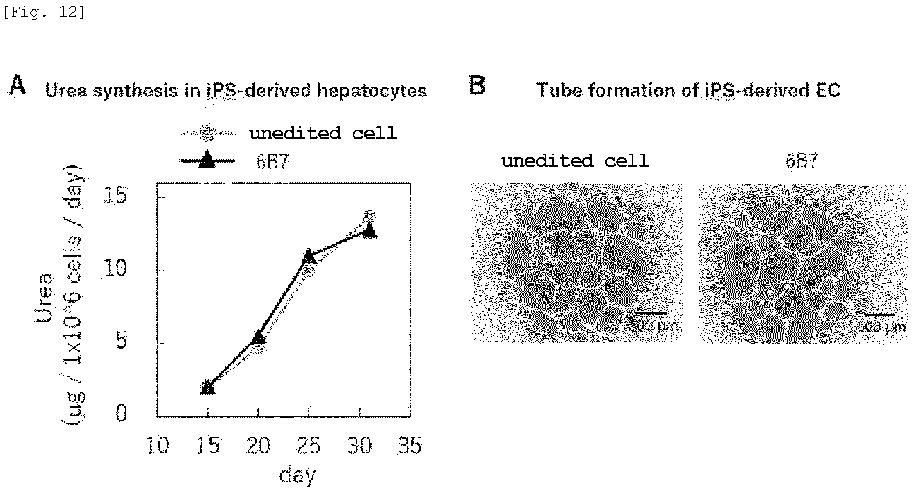

Differentiation of iPS cells into hepatocytes was performed according to the literatures (PNAS. 2012 Jul 31; 109(31):12538-43 (up to DE induction in the first half), and Hepatology, 2010 Jan; 51(1): 297-305 and Stembook, Cai J. et al., Protocol for directed differentiation of human pluripotent stem cells toward a hepatocyte fate (using the latter HCM)). As an evaluation of the differentiation potential into hepatocytes, the urea synthesis ability, which is one of liver-specific functions, was evaluated (Fig. 12A). As a result, the urea synthesis ability of clone 6B7 was almost equivalent to that of unedited iPS cell clone 06E.

Differentiation of iPS cells into vascular endothelial cells was performed according to the literature (Nat Cell Biol. 2015 Aug; 17(8): 994-1003). As intravascular cell markers, anti-CD31 antibody (clone WM59, BD PharmingenD, 555445) and anti-CD144 antibody (clone 55-7H1, BD Pharmingen, 560410) were used. As one of the evaluations of the differentiation potential into intravascular cells, angiopoietic potential was evaluated (Fig. 12B). As a result, the angiopoietic potential of clone 6B7 was almost equivalent to that of unedited iPS cell clone 06E.

From the above results, it was confirmed that the differentiation potential of the HLA class Ia&II-deficient iPS cell clone 6B7 is of the same level as that of the unedited iPS cell clone 06E.

### [Example 3] Production of universal donor iPS cell

### <Forced expression of gene that enhances immune evasion function>

In order to suppress immunorejection by T cells and NK cells, universal donor iPS cells were produced by forcibly expressing the following factors in the HLA class Ia&II deficient iPS cell clone 6B7 obtained in Example 2. As the gene transfer method, the PiggyBac method was used. Constitutively active EF1alpha promoter region was used for cDNA forced expression of each factor.

As vectors used for gene transfer by the PiggyBac method, the following vectors were prepared. PD-L1 and PD-L2 were linked by the P2A sequence and expressed simultaneously in one vector.
#PB_PD-L1_P2A_PD-L2_Puro_Vector
#PB_B2M_Puro_Vector
#PB_HLAG_Puro_Vector
#PB_iCasp9_Puro_Vector

In addition, the following vector was prepared as a PiggyBac transposase.
#hPBase_Hygro_Vector

Each vector used for gene transfer by the PiggyBac method was constructed by the following method. PiggyBac 3'ITR sequence (SEQ ID NO: 13)-restriction enzyme MCS-PiggyBac 5'ITR sequence (SEQ ID NO: 14) was artificially synthesized and inserted into the PstI and EcoRI restriction enzyme sites of pHSG298 plasmid. PB_CDS_Puro_Vector expressing each target gene was constructed by inserting EF1A promoter (amplified from pBApo-EF1a Pur DNA plasmid by PCR), each target gene (PD-L1-P2A-PD-L2:SEQ ID NO: 15, HLAG:SEQ ID NO: 16, B2M:SEQ ID NO: 17, iCasp9 CDS (artificial synthesis):SEQ ID NO: 19), and IRES-Puro-hGHpolyA (artificial synthesis):SEQ ID NO: 23 into MCS.

In addition, hPBase_Hygro_Vector was constructed by the following method. hPBase vector was constructed by inserting EF1A promoter (amplified by PCR), Human codon-optimized PBase (artificial synthesis, SEQ ID NO: 18), and IRES-Hygro-hGHpolyA (artificial synthesis):SEQ ID NO: 24 into the SalI and KpnI restriction enzyme sites of pHSG298 plasmid.

Then, the above-mentioned 5 kinds of plasmid DNAs were introduced into the HLA class Ia&II-deficient iPS cell clone 6B7 obtained in Example 2 by using an electroporation method (Neon Transfection System, Thermo Fisher Scientific). The cells transfected here were named "HGEC-0012 cells".

The medium was exchanged with a liquid medium containing Puromycin and Hygromycin the next day after introduction of the plasmid DNA, and the selection of drug-resistant cells was started. From 2 days to 5 days after electroporation, the medium was replaced with a Puromycin-containing liquid medium every day and drug selection was continued. Six days after electroporation, the medium was replaced with a drug-free liquid medium, and cell culture was continued. Seven days after the electroporation, the cells were seeded such that each well of a 96-well plate contained one cell as a calculated value, and culture was continued. Twelve days after seeding on the 96-well plate, the cells were passaged in each well of a 24-well plate and a 96-well plate. The cells on the 96-well plate after 12+1 days from the seeding in the 96-well plate such that one cell was contained per well as a calculated value were collected the day after passage, the expression level of each introduced factor was analyzed by the quantify RT-PCR method, and clones overexpressing all introduced factors were screened.

Primary screening by quantitative RT-PCR was performed by the following method. A reverse transcription reaction was performed to analyze cells on the 96-well plate (SuperPrep Cell Lysis and RT Kit for qPCR, TOYOBO, SCQ-401). Using this reverse transcription product as a template, quantitative PCR (StepOne Plus real-time PCR system, ThermoFisher Scientific) was performed to analyze the expression level of the introduced gene. Either reagent of THUNDERBIRD Probe qPCR Mix (TOYOBO, QPS-101) or TaqMan Fast Advanced Master Mix (ThermoFisher Scientific, 4444557) was used for the PCR reaction. TaqMan Gene Expression Assay (FAM and/or VIC) (ThermoFisher Scientific) was used to detect each factor; GAPDH (Hs02758991_g1), PD-L1 (Hs00204257_m1), PD-L2 (Hs00228839_m1), HLAG (Hs00365950_g1), B2M (Hs00187842_m1). Fluorescent probes and primers for detecting iCasp9 were artificially synthesized (see below for sequences).

The artificially synthesized fluorescent probe (FAM) and primer sequences are shown below.
#iCasp9-fwd: GAACTGCTGAAGCTGGAATC(SEQ ID NO: 20)
#iCasp9-rev: CATTTCCTCTCAGGCTTTCCAG(SEQ ID NO: 21)
#iCasp9-probe(5'6-FAM, 3'BHQ1):
ATCTGGCGTTGACGGCTTTGGAGATGTG(SEQ ID NO: 22)

Single-cell-derived 242 clones were isolated from twenty 96-well plates, and one clone was seeded in two wells of "24-well plate for continuous culture" and "96-well plate for screening". The cells of the "96-well plate for screening" were collected one day after seeding and analyzed by the above-mentioned reverse transcription quantitative PCR method. As a result, overexpression of PD-L1, HLA-G, B2M and iCasp9 mRNAs was confirmed in 18 clones. As a comparison target, an HLA class Ia&II-deficient iPS cell clone 6B7 without gene transfer was used. On the other hand, the cells seeded in the "24-well plate for continuous culture" were passaged into two 9 cm dishes only for the above-mentioned 18 clones one week after seeding. Six days after seeding, secondary screening was performed by quantifying protein surface expression by a flow cytometry method using cells from one 9 cm dish. The cells in the remaining 9 cm dish were cryopreserved after culturing.

Secondary screening by the flow cytometry method was performed according to the following method. After detaching the cells from the dish, the cell suspension was mixed with a fluorescence-labeled antibody, and the expression levels of proteins expressed on the cell surface were quantitatively analyzed using a FACSVerse flow cytometer (BD). The antibodies used are indicated below; anti-PD-L1 antibody-APC labeled (clone MIH1, Invitrogen, 12-5888-42), anti-B2M antibody-PECy7 labeled (clone 2M2, BioLegend, 316318), anti-HLAG antibody-PE labeled (clone MEM-G/9, Abcam, ab24384). As a result, high expression of PD-L1, HLA-G, and B2M on the cell surface was confirmed in all 18 clones. Universal donor iPS cell clones were isolated by the above-mentioned method.

### «Evaluation of obtained cells»

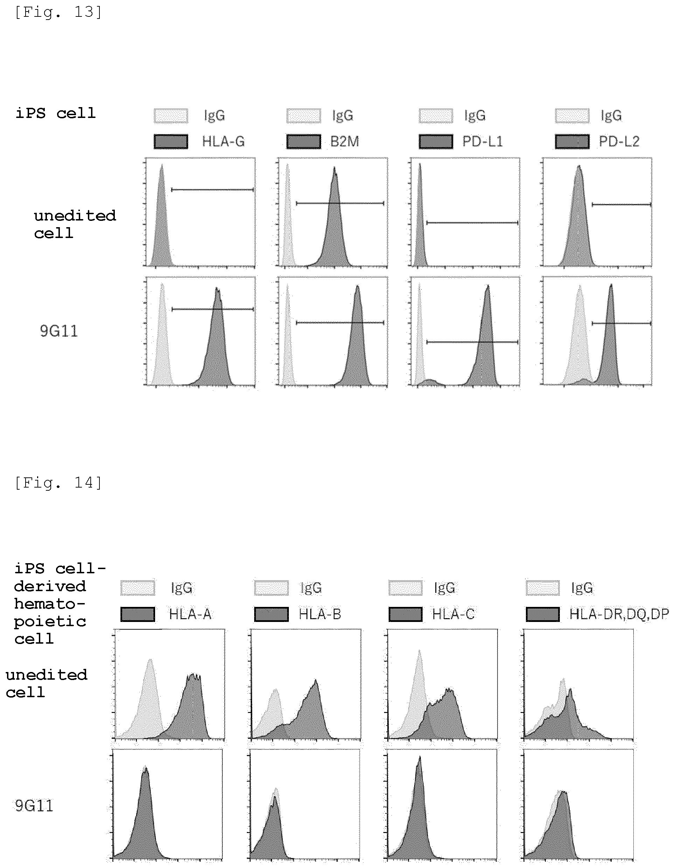

The overexpression levels of HLA-G, B2M, PD-L1, and PD-L2 on the cell surface in the universal donor iPS cell clone 9G11 were confirmed by a method similar to the above-mentioned method. The antibodies used are described below; anti-PD-L1 antibody-APC labeled (clone MIH1, Invitrogen, 12-5888-42), anti-PD-L2 antibody-PE labeled (clone MIH18, Invitrogen, 17-5983-42), anti-HLAG antibody-PE labeled (clone MEM-G/9, Abcam, ab24384). As a result, high expression of HLA-G, B2M, PD-L1, and PD-L2 on the cell surface was confirmed (Fig. 13).

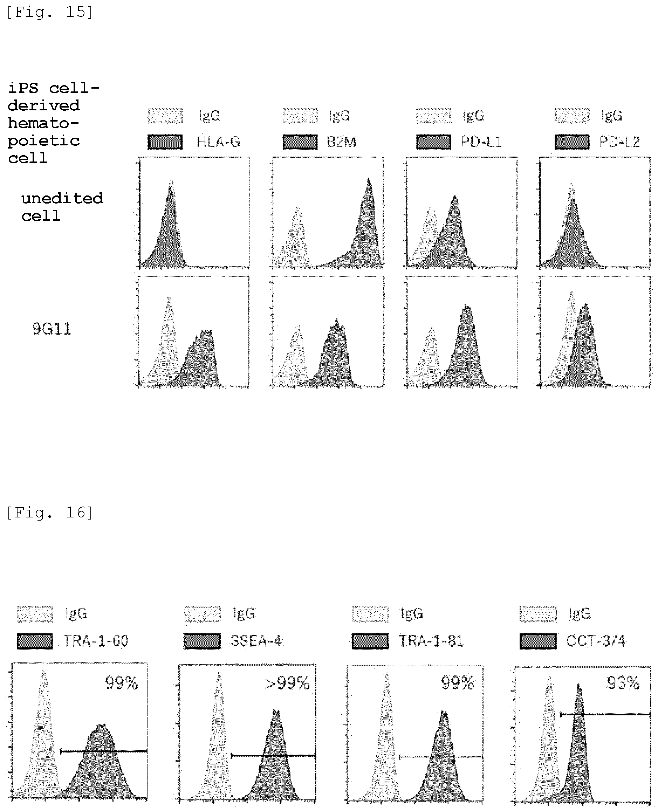

After differentiation of the universal donor iPS cell clone 9G11 into a hematopoietic cell population (CD45-positive cells) containing hematopoietic progenitor cells, the cell surface expression of HLA class Ia protein was analyzed by a flow cytometry method in the same manner as in Example 1, and the expression level was confirmed. Differentiation of the universal donor iPS cell into the hematopoietic progenitor cell was performed in the same manner as in Example 2. As a result, a drastic decrease in the cell surface expression level of HLA-A, HLA-B, and HLA-C proteins was confirmed (Fig. 14). In addition, the cell surface expression level of HLA class II protein was analyzed by a flow cytometry method in the same manner as in Example 2. As a result, a drastic decrease in the cell surface expression levels of HLA class II proteins (HLA-DR, DP, DQ) was confirmed (Fig. 14). Furthermore, the overexpression levels of HLA-G, PD-L1, and PD-L2 on the cell surface were confirmed by a method similar to the above-mentioned method. The antibodies used are described below; anti-PD-L1 antibody-APC labeled (clone MIH1, Invitrogen, 12-5888-42), anti-PD-L2 antibody-PE labeled (clone MIH18, Invitrogen, 17-5983-42), anti-B2M antibody-PECy7 labeled (clone 2M2, BioLegend, 316318), anti-HLAG antibody-PE labeled (clone MEM-G/9, Abcam, ab24384). As a result, high expression of HLA-G on the cell surface was confirmed. As for PD-L1 and PD-L2, although endogenous expression was also observed, a slight increase in the expression was confirmed (Fig. 15).

In order to confirm whether the forced expression of HLA-G, B2M, PD-L1, PD-L2, and iCasp9 in addition to the deficiency of HLA-A, HLA-B, HLA-C, and RFXANK proteins caused changes in the undifferentiated state of iPS cells, expression of iPS cell undifferentiated markers in universal donor iPS cell clone 9G11 was analyzed by a flow cytometry method as in Example 1. AntiTRA-1-81 antibody (clone TRA-1-81), antiSSEA-4 antibody (clone MC813-70), and antiTRA-1-60 antibody (clone TRA-1-60) were used as antibodies against undifferentiated markers on the cell surface. In addition, expression of intracellular Oct3/4 protein was analyzed by a flow cytometry method. In order to stain intracellular proteins, the cell suspension was treated with Permeabilization Buffer (BD) and then stained with an anti-Oct3/4 antibody (clone C30A3). As a result, it was confirmed that universal donor iPS cell clone 9G11 firmly expressed each undifferentiated marker (Fig. 16). The expression of undifferentiated marker was also confirmed by an immunostaining method. Furthermore, it was confirmed by an alkaline phosphatase staining method that the undifferentiated state was not affected.

In order to confirm that the gene expression pattern did not change significantly before and after gene editing, an RNA-seq comparison analysis was performed using the universal donor iPS cell clone 9G11 and unedited iPS cells. As a result, it was confirmed that the gene expression pattern did not change significantly before and after gene editing.

In addition, in order to examine the presence or absence of known gene mutation that may increase canceration risk during the culture process for gene editing and cloning, highly sensitive genome mutation analysis using the QIAseq Targeted DNA Panel (Comprehensive Cancer Panel) was performed. As a result, it was confirmed that no known mutation known to increase the risk of canceration occurred during the culture process for gene editing and cloning.

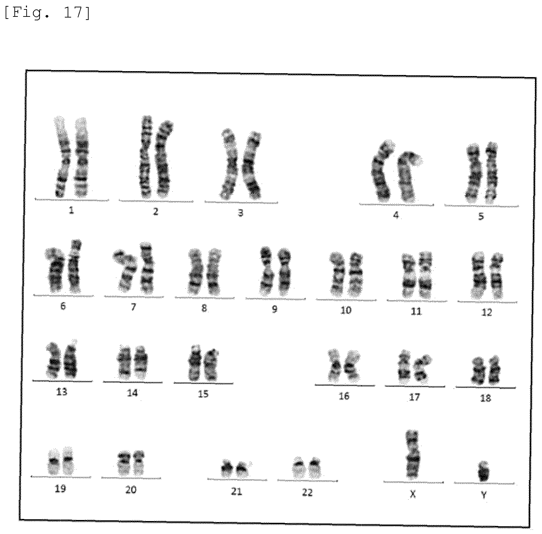

In order to examine the presence or absence of karyotype abnormality during the culture process for gene editing and cloning, karyotype analysis was performed. As a result, karyotype abnormality was not detected in the universal donor iPS cell clone 9G11 (Fig. 17).

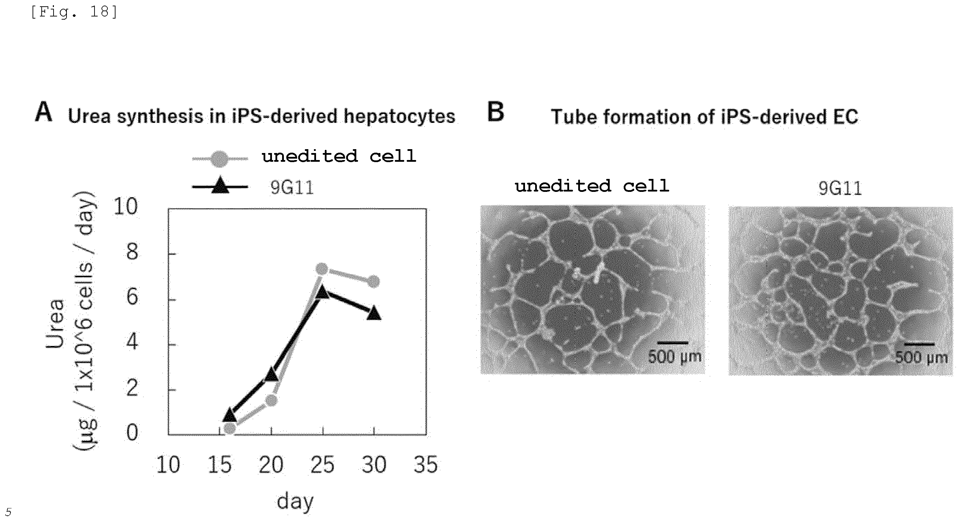

In order to confirm whether the forced expression of HLA-G, B2M, PD-L1, PD-L2, and iCasp9 in addition to the deficiency of HLA-A, HLA-B, HLA-C, and RFXANK proteins caused changes in the maintenance of differentiation potential of iPS cells into multiple lineages, induction of the differentiation of the universal donor iPS cell clone 9G11 into multiple lineages was tried. Maintenance of pluripotency was confirmed by a method similar to that in Example 2 (Fig. 18).

### Example 4: Evaluation of immune evasion mechanism and safety mechanism of universal donor iPS cells

### <<Response of T cell>>

Universal donor iPS cell clones are expected to easily avoid immune responses by T cells as a result of gene editing. In order to confirm that the response of T cell caused by HLA mismatch was attenuated against the universal donor iPS cell clone 9G11, analysis by a flow cytometry method was performed using the proliferation of T cells as an index. As target cells, a cell population (CD45-positive cells) containing hematopoietic progenitor cells derived from universal donor iPS cells (clone 9G11) was used. Differentiation of the universal donor iPS cells into hematopoietic progenitor cells was performed in the same manner as in Example 2. T cells and target cells were cocultured for 5 or 7 days, and the proliferation of the T cells was evaluated using EdU reagent. For T cells, the EdU-positive rate of each of CD4-positive cells and CD8-positive cells was calculated as the proliferation rate of each cell. As a positive control of the test system, the proliferation of T cells was evaluated using THP-1 cells as the target cells.

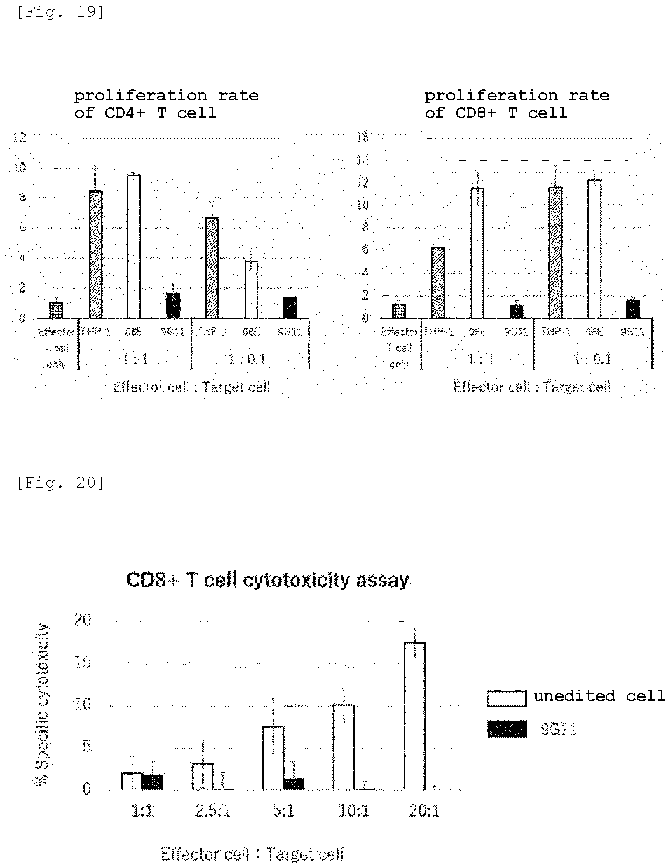

The results are shown in Fig. 19. Cell proliferation was hardly observed with T cells alone (not incorporating EdU). When co-cultured with positive control THP-1 or unedited iPS cell-derived CD45-positive cells (06E), T cells responded and showed cell proliferation (incorporating EdU). In contrast, the universal donor iPS cell clone 9G11 showed a marked decrease in the response of T cells (decreased EdU uptake) when cocultured. From these results, it was confirmed that, by gene editing operation, the differentiated cells derived from universal donor iPS cell clone more easily avoided immune responses by T cells.

### <<Avoidance of cytotoxic activity by T cells>>

In order to confirm that the injury activity of T cells against the universal donor iPS cell clone 9G11 was attenuated, a cytotoxicity assay was performed by the flow cytometry method. CD8-positive cell was used as the effector cell, and a cell population (CD45-positive cells) containing hematopoietic progenitor cells derived from iPS cells (clone 9G11 and unedited cells) was used as the target cell. CD45-positive cells were cultured in the presence of 50 ng/mL IFNγ for 2 days, and viable cells were stained using Cell Tracer Violet (Thermo Fisher Scientific) at a final concentration of 5 µM. The primed CD8-positive cells were mixed at different cell number ratios and cocultured for 3 hr, after which 250 µL of 0.1 µM SYTOX solution of SYTOX, a dead cell marker, was added to label injured target cells. Thereafter, the fluorescence of Cell Tracer Violet and SYTOX was measured with a flow cytometer (Miltenyi Biotec MACSQuant) .

The percentage of dead cells (SYTOX positive) in all target cells (Cell Tracer Violet positive) was measured and used as an index of cytotoxic activity. Unprimed T cells were tested as a negative control reaction and subtracted as background values.

As a result, the unedited cells were damaged by the primed CD8-positive cells, whereas the universal donor iPS cell clone 9G11 was confirmed to be less susceptible to damage, as expected (Fig. 20) .

### <<Avoidance of cytotoxic activity by NK cells>>

Then, the cytotoxic activity of NK cells against iPS cells was evaluated by the following method. It is expected that, as a result of gene editing, universal donor iPS cell clones easily avoid immune response by NK cells. In order to confirm that the response of NK cells to the universal donor iPS cell clone 9G11 is attenuated, analysis by a flow cytometry method was performed using the proliferation of NK cell as an index. As target cells, unedited iPS cell clone 06E; HLA class Ia&II-deficient iPS cell clone 6B7; and universal donor iPS cell clone 9G11 were used.

In order to label the target cells with a viable cell marker calcein, 0.03 mM Calcein-AM solution was added to the target cell suspension (100,000 cells/100 µL) at a final concentration of 500 nM for K562, and at a final concentration of 1500 nM for iPS cells. The cells were allowed to stand in a 5% CO₂ incubator at 37°C for 30 min. 5 mL of PBS was added, and the mixture was centrifuged at 200xg for 4 min, and the supernatant was removed. 250,000 or 500,000 effector cells (NK cells) and 50,000 target cells were mixed and allowed to stand in a 5% CO₂ incubator at 37°C for 2 hr. 0.25% BSA-PBS solution was added, and the mixture was centrifuged at 300xg for 5 min, and the supernatant was removed. Then, 250 µL of 0.1 nM SYTOX solution, which is a dead cell marker, was added to label injured target cells. Thereafter, the fluorescence of calcein and SYTOX was measured with a flow cytometer (Miltenyi Biotec MACSQuant). The percentage of dead cells (SYTOX-positive) in all target cells (calcein-positive) was measured and used as an index of cytotoxic activity. An appropriate negative control reaction was performed to calculate numerical values and they were subtracted as background values.

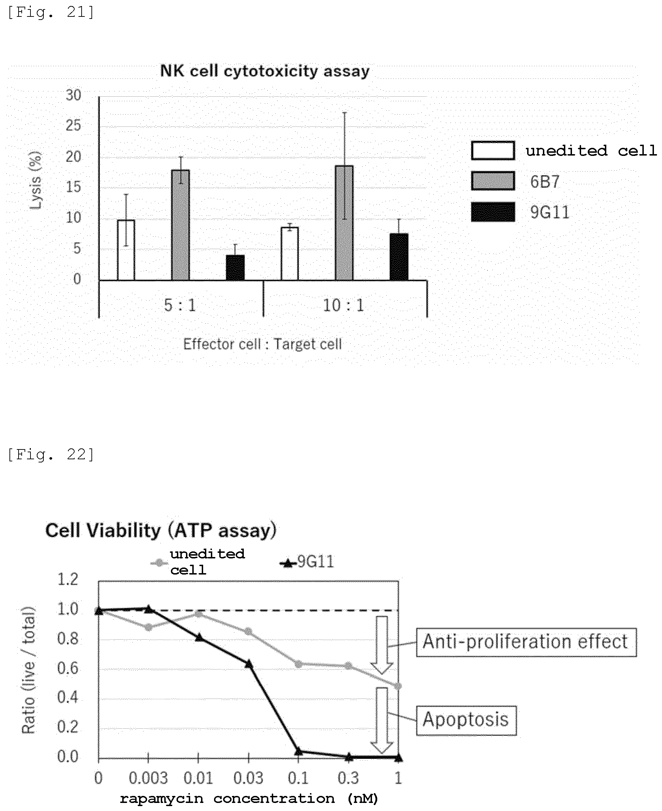

As a measurement result of the cytotoxic activity by NK cells, HLA class Ia&II deficient iPS cell clone 6B7 hardly expressed HLA class I protein on the cell surface compared to unedited iPS cells expressing HLA class I protein on the cell surface, as described above, and, as expected, more cells were injured by NK cells than unedited iPS cells. In the universal donor iPS cell clone 9G11, the forced expression of HLA-G and B2M resulted in the expression of a large amount of HLA class I protein on the cell surface as described above, and, as expected, only the cells at the same level as or a lower level than that of unedited iPS cells were injured by NK cells (Fig. 21). Therefore, it was clarified that the cells of the present invention have a high ability to avoid the cytotoxic activity of NK cells.

### <<Functional evaluation of suicide genes>>

Next, an experiment was conducted to confirm whether the introduced suicide gene is functionable. In order to confirm the function of the suicide gene iCaspase9 introduced into universal donor iPS cells as a safety mechanism, the cell death induction by the addition of rapamycin in vitro was confirmed. In order to evaluate the cell death induction efficiency, CellTiter Glo Assay Kit (Promega) was used to luminescence measure the cell viability based on the amount of ATP, which is an index of cell viability. The above-mentioned iCaspase9-introduced cells (clone B), and iCaspase9-free unedited cells (Parental) were cultured for 24 hr under the condition of each rapamycin concentration (0 nM, 0.003 nM, 0.01 nM, 0.03 nM, 0.1 nM, 0.3 nM, 1 nM) and the viable cell rate was measured. As a result, it was clarified that the cell viability of the iCaspase9-introduced cells markedly decreased compared with the unedited cells at a rapamycin concentration of 0.1 nM or higher (Fig. 22). From these results, it was confirmed that the cell death was induced by iCaspase9 under the condition of rapamycin concentration of 0.1 nM or higher.

### [Example 5] Expression of HLA class Ib in the presence or absence of B2M

In order to examine whether the exogenous B2M introduced into the cells of the present invention in Example 3 is necessary for the production of the cells of the present invention, a comparison test was conducted to see whether or not the expression of HLA class Ib was maintained when the exogenous B2M was removed.
"combination 1"
   #PB_B2M_Puro_Vector
   #PB_HLAG_Puro_Vector
"combination 2"
   #PB_HLAG_Puro_Vector

A PiggyBac gene expression vectors of the above-mentioned "combination 1" or "combination 2" was introduced by the electroporation method into the HLA class Ia&II-deficient iPS cells obtained in Example 2. Drug selection was performed according to the method described in Example 3. After 6 days from the electroporation, the medium was replaced with a drug-free liquid medium, and cell culture was continued. Cells in this state are hereinafter referred to as "pool cells".

The cell surface expression of forced expression factors was analyzed by a flow cytometry method. The cells were detached from the dish, mixed with fluorescently labeled antibodies, and the expression levels of the proteins expressed on the cell surface were quantitatively analyzed using a flow cytometer (BD FACSVerse). The antibodies used are described below; anti-B2M antibody-PECy7 labeled (clone 2M2, BioLegend, 316318), anti-HLA-G antibody-PE labeled (clone MEM-G/9, Abcam, ab24384).

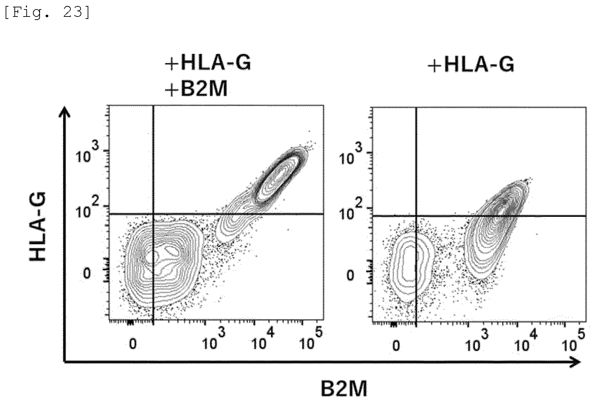

The above-mentioned two types of combinations were compared in the pooled cells. As a result, higher levels of HLA-G and B2M expression on the cell surface were confirmed by introducing B2M (combination 1) (Fig. 23). Since the total amount of HLA class I (presumed from cell surface expression of B2M) increases in the presence of B2M, it was expected that introduction of B2M would make the cells of the present invention more highly functional.

### [Industrial Applicability]

The cells of the present disclosure are useful, for example, in the field of regenerative medicine. This application is based on a patent application No. 2020-091787 filed in Japan (filing date: May 26, 2020), the contents of which are incorporated in full herein.

## Claims

1. A hypoimmunogenic human cell
(1) lacking an endogenous gene encoding an α chain of human leukocyte antigen (HLA) class Ia,
(2) lacking an endogenous gene encoding HLA class II or an expression regulator thereof,
(3) comprising an exogenous gene encoding an α chain of HLA class Ib,
(4) comprising an exogenous gene encoding human PD-L1, and
(5) comprising an exogenous gene encoding human PD-L2.

2. The cell according to claim 1, wherein the cell does not express endogenous HLA class Ib on a cell surface.

3. The cell according to claim 1 or 2, wherein the endogenous gene encoding the α chain of the HLA class Ia comprises an endogenous gene encoding an α chain of HLA-A, an endogenous gene encoding an α chain of HLA-B, and an endogenous gene encoding an α chain of HLA-C.

4. The cell according to any one of claims 1 to 3, wherein the endogenous gene encoding the HLA class II or an expression regulator thereof comprises the following (a) or (b):
(a) an endogenous gene encoding an α chain and/or a β chain of HLA-DP, an endogenous gene encoding an α chain and/or a β chain of HLA-DQ, an endogenous gene encoding an α chain and/or a β chain of HLA-DR, an endogenous gene encoding an α chain and/or a β chain of HLA-DM, and an endogenous gene encoding an α chain and/or a β chain of HLA-DO,
(b) an endogenous gene encoding human RFXANK, an endogenous gene encoding human RFX5, an endogenous gene encoding human RFXAP, and an endogenous gene encoding human CIITA.

5. The cell according to any one of claims 1 to 4, wherein the exogenous gene encoding the α chain of the HLA class Ib comprises an exogenous gene encoding an α chain of HLA-E and/or an exogenous gene encoding an α chain of HLA-G.

6. The cell according to any one of claims 1 to 5, comprising (6) an exogenous gene encoding human β2 microglobulin.

7. The cell according to any one of claims 1 to 6, comprising (7) a suicide gene.

8. The cell according to any one of claims 1 to 7, wherein the site containing the exogenous gene or suicide gene is a safe harbor region of the genome.

9. The cell according to claim 8, wherein the safe harbor region is an AAVS1 region, a CCR5 region, or a ROSA26 region.

10. The cell according to any one of claims 1 to 9, wherein the hypoimmunogenic human cell is a pluripotent stem cell or a differentiated cell thereof.

11. A method for producing a hypoimmunogenic human cell, comprising the following steps:
(i) a step of deleting an endogenous gene encoding an α chain of HLA class Ia of a human parental cell,
(ii) a step of deleting an endogenous gene encoding HLA class II or an expression regulator thereof from the human parental cell,
(iii) a step of introducing an exogenous gene encoding an α chain of HLA class Ib into the human parental cell,
(iv) a step of introducing an exogenous gene encoding human PD-L1 into the human parental cell, and
(v) a step of introducing an exogenous gene encoding human PD-L2 into the human parental cell.

12. The method according to claim 11, wherein the hypoimmunogenic human cell does not express endogenous HLA class Ib on a cell surface.

13. The method according to claim 11 or 12, wherein the endogenous gene encoding the α chain of the HLA class Ia comprises an endogenous gene encoding an α chain of HLA-A, an endogenous gene encoding an α chain of HLA-B, and an endogenous gene encoding an α chain of HLA-C.

14. The method according to any one of claims 11 to 13, wherein the endogenous gene encoding HLA class II or an expression regulator thereof comprises the following (a) or (b) :
(a) an endogenous gene encoding an α chain and/or a β chain of HLA-DP, an endogenous gene encoding an α chain and/or a β chain of HLA-DQ, an endogenous gene encoding an α chain and/or a β chain of HLA-DR, an endogenous gene encoding an α chain and/or a β chain of HLA-DM, and an endogenous gene encoding an α chain and/or a β chain of HLA-DO,
(b) an endogenous gene encoding human RFXANK, an endogenous gene encoding human RFX5, an endogenous gene encoding human RFXAP, and an endogenous gene encoding human CIITA.

15. The method according to any one of claims 11 to 14, wherein the exogenous gene encoding the α chain of the HLA class Ib comprises an exogenous gene encoding an α chain of HLA-E and/or an exogenous gene encoding an α chain of HLA-G.

16. The method according to any one of claims 11 to 15, further comprising the following step:
(vi) a step of introducing an exogenous gene encoding human β2 microglobulin into the human parental cell.

17. The method according to any one of claims 11 to 16, further comprising the following step:
(vii) a step of introducing a suicide gene into the human parental cell.

18. The method according to any one of claims 11 to 17, wherein the site into which the exogenous gene or suicide gene is introduced is a safe harbor region of the genome.

19. The method according to claim 18, wherein the safe harbor region is an AAVS1 region, a CCR5 region, or a ROSA26 region.

20. The method according to any one of claims 11 to 19, wherein the human parental cell is a pluripotent stem cell or a differentiated cell thereof.
